# EUROPEAN PATENT APPLICATION

(11) **EP 1 227 092 A2**
(43) Date of publication of application: **31.07.2002**
(21) Application number: 02076582.2
(22) Date of filing: 15.07.1994
(51) Int. Cl.: C07D 403/04, C07D 401/04, C07C 317/28, C07C 321/28

(54) **Isonitrile intermediates for the preparation of tri-substituted imidazole compounds with multiple therapeutic properties**

(30) Priority: 16.07.1993 US 92733
(62) Divisional of application: 94923503.0
(71) Applicant: SmithKline Beecham Corporation, Philadelphia, PA 19103 (US)
(72) Inventor: Adams, Jerry Leroy, Wayne, PA 19087 (US); Sheldrake, Peter William, Turnbridge Wells, Kent TN2 7NW (GB); Gallagher, Timothy Francis, Harleysville, PA 19438 (US); Garigipati, Ravi Shanker, Wayne, PA 19087 (US); Bender, Paul Elliot, Cherry Hill, NJ 08003 (US); Boehm, Jeffrey Charles, King of Prussia, PA 19406 (US)
(74) Representative: West, Vivien

(57) **Abstract**

The invention relates to a novel group of isonitrile compounds and a process for their preparation, useful in the preparation of tri-substituted imadazoles having multiple therapeutic properties.

## Description

This invention relates to a novel group of imidazole compounds, processes for the preparation thereof, the use thereof in treating cytokine mediated diseases and pharmaceutical compositions for use in such therapy.

### BACKGROUND OF THE INVENTION

Interleukin-1 (IL-1) and Tumor Necrosis Factor (TNF) are biological substances produced by a variety of cells, such as monocytes or macrophages. IL-1 has been demonstrated to mediate a variety of biological activities thought to be important in immunoregulation and other physiological conditions such as inflammation [See, e.g., Dinarello et al., Rev. Infect. Disease, 6, 51 (1984)]. The myriad of known biological activities of IL-1 include the activation of T helper cells, induction of fever, stimulation of prostaglandin or collagenase production, neutrophil chemotaxis, induction of acute phase proteins and the suppression of plasma iron levels.

There are many disease states in which excessive or unregulated IL-1 production is implicated in exacerbating and/or causing the disease. These include rheumatoid arthritis, osteoarthritis, endotoxemia and/or toxic shock syndrome, other acute or chronic inflammatory disease states such as the inflammatory reaction induced by endotoxin or inflammatory bowel disease; tuberculosis, atherosclerosis, muscle degeneration, cachexia, psoriatic arthritis, Reiter's syndrome, rheumatoid arthritis, gout, traumatic arthritis, rubella arthritis, and acute synovitis. Recent evidence also links IL-1 activity to diabetes and pancreatic β cells.

Dinarello, J. Clinical Immunology, 5 (5), 287-297 (1985), reviews the biological activities which have been attributed to IL-1. It should be noted that some of these effects have been described by others as indirect effects of IL-1.

Excessive or unregulated TNF production has been implicated in mediating or exacerbating a number of diseases including rheumatoid arthritis, rheumatoid spondylitis, osteoarthritis, gouty arthritis and other arthritic conditions; sepsis, septic shock, endotoxic shock, gram negative sepsis, toxic shock syndrome, adult respiratory distress syndrome, cerebral malaria, chronic pulmonary inflammatory disease, silicosis, pulmonary sarcoisosis, bone resorption diseases, reperfusion injury, graft vs. host reaction, allograft rejections, fever and myalgias due to infection, such as influenza, cachexia secondary to infection or malignancy, cachexia, secondary to acquired immune deficiency syndrome (AIDS), AIDS, ARC (AIDS related complex), keloid formation, scar tissue formation, Crohn's disease, ulcerative colitis, or pyresis.

AIDS results from the infection of T lymphocytes with Human Immunodeficiency Virus (HIV). At least three types or strains of HIV have been identified, i.e., HIV-1, HIV-2 and HIV-3. As a consequence of HIV infection, T-cell mediated immunity is impaired and infected individuals manifest severe opportunistic infections and/or unusual neoplasms. HIV entry into the T lymphocyte requires T lymphocyte activation. Other viruses, such as HIV-1, HIV-2 infect T lymphocytes after T Cell activation and such virus protein expression and/or replication is mediated or maintained by such T cell activation. Once an activated T lymphocyte is infected with HIV, the T lymphocyte must continue to be maintained in an activated state to permit HIV gene expression and/or HIV replication. Monokines, specifically TNF, are implicated in activated T-cell mediated HIV protein expression and/or virus replication by playing a role in maintaining T lymphocyte activation. Therefore, interference with monokine activity such as by inhibition of monokine production, notably TNF, in an HIV-infected individual aids in limiting the maintenance of T cell activation, thereby reducing the progression of HIV infectivity to previously uninfected cells which results in a slowing or elimination of the progression of immune dysfunction caused by HIV infection. Monocytes, macrophages, and related cells, such as kupffer and glial cells, have also been implicated in maintenance of the HIV infection. These cells, like T-cells, are targets for viral replication and the level of viral replication is dependent upon the activation state of the cells. [See Rosenberg et al., The Immunopathogenesis of HIV Infection, Advances in Immunology, Vol. 57, (1989)]. Monokines, such as TNF, have been shown to activate HIV replication in monocytes and/or macrophages [See Poli, et al., Proc. Natl. Acad. Sci., 87:782-784 (1990)], therefore, inhibition of monokine production or activity aids in limiting HIV progression as stated above for T-cells.

TNF has also been implicated in various roles with other viral infections, such as the cytomegalia virus (CMV), influenza virus, and the herpes virus for similar reasons as those noted.

Interleukin-8 (IL-8) is a chemotactic factor first identified and characterized in 1987. IL-8 is produced by several cell types including mononuclear cells, fibroblasts, endothelial cells, and keratinocytes. Its production from endothelial cells is induced by IL-1, TNF, or lipopolysachharide (LPS). Human IL-8 has been shown to act on Mouse, Guinea Pig, Rat, and Rabbit Neutrophils. Many different names have been applied to IL-8, such as neutrophil attractant/activation protein-1 (NAP-1), monocyte derived neutrophil chemotactic factor (MDNCF), neutrophil activating factor (NAF), and T-cell lymphocyte chemotactic factor.

IL-8 stimulates a number of functions in vitro. It has been shown to have chemoattractant properties for neutrophils, T-lymphocytes, and basophils. In addition it induces histamine release from basophils from both normal and atopic individuals as well as lysozomal enzyme release and respiratory burst from neutrophils. IL-8 has also been shown to increase the surface expression of Mac-1 (CD11b/CD18) on neutrophils without de novo protein synthesis, this may contribute to increased adhesion of the neutrophils to vascular endothelial cells. Many diseases are characterized by massive neutrophil infiltration. Conditions associated with an increased in IL-8 production (which is responsible for chemotaxis of neutrophil into the inflammatory site) would benefit by compounds which are suppressive of IL-8 production.

IL-1 and TNF affect a wide variety of cells and tissues and these cytokines as well as other leukocyte derived cytokines are important and critical inflammatory mediators of a wide variety of disease states and conditions. The inhibition of these cytokines is of benefit in controlling, reducing and alleviating many of these disease states.

There remains a need for treatment, in this field, for compounds which are cytokine suppressive anti-inflammatory drugs, i.e. compounds which are capable of inhibiting cytokines, such as IL-1, IL-6, IL-8 and TNF.

### SUMMARY OF THE INVENTION

This invention relates to the novel compounds of Formula (I) and pharmaceutical compositions comprising a compound of Formula (I) and a pharmaceutically acceptable diluent or carrier.

This invention also relates to a method of inhibiting cytokines and the treatment of a cytokine mediated disease, in a mammal in need thereof, which comprises administering to said mammal an effective amount of a compound of Formula (I).

This invention more specifically relates to a method of inhibiting the production of IL-1 in a mammal in need thereof which comprises administering to said mammal an effective amount of a compound of Formula (I).

This invention more specifically relates to a method of inhibiting the production of IL-8 in a mammal in need thereof which comprises administering to said mammal an effective amount of a compound of Formula (I).

This invention more specifically relates to a method of inhibiting the production of TNF in a mammal in need thereof which comprises administering to said mammal an effective amount of a compound of Formula (I).

Accordingly, the present invention provides a compound of Formula (I): 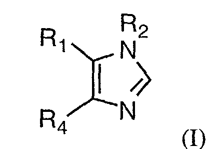
- R₁: is 4-pyridyl, pyrimidinyl, quinolyl, isoquinolinyl, quinazolin-4-yl, 1-imidazolyl or 1-benzimidazolyl, which heteroaryl ring is optionally substituted with one or two substituents each of which is independently selected from C₁₋₄ alkyl, halogen, hydroxyl, C₁₋₄ alkoxy, C₁₋₄ alkylthio, C₁₋₄ alkylsulfinyl, CH₂OR₁₂, NR₁₀R₂₀, or an N-heterocyclyl ring which ring has from 5 to 7 members and optionally contains an additional heteroatom selected from oxygen, sulfur or NR₁₅;
- R₄: is phenyl, naphth-1-yl or naphth-2-yl, or a heteroaryl, which is optionally substituted by one or two substituents, each of which is independently selected, and which, for a 4-phenyl, 4-naphth-1-yl, 5-naphth-2-yl or 6-naphth-2-yl substitiuent, is halogen, cyano, nitro, -C(Z)NR₇R₁₇, -C(Z)OR₁₆, -(CR₁₀R₂₀)ₘCOR₁₂, -SR₅, -SOR₅, -OR₁₂, halo-substituted-C₁₋₄ alkyl, C₁₋₄ alkyl, -ZC(Z)R₁₂, -NR₁₀C(Z)R₁₆, or -(CR₁₀R₂₀)ₘNR₁₀R₂₀ and which, for other positions of substitution, is halo, cyano, -C(Z)NR₁₃R₁₄, -C(Z)OR₃, -(CR₁₀R₂₀)ₘCOR₃, -S(O)ₘR₃, -OR₃, halo-substituted-C₁₋₄ alkyl, -C₁₋₄ alkyl, -(CR₁₀R₂₀)ₘNR₁₀C(Z)R₃, -NR₁₀S(O)ₘR₈, -NR₁₀S(O)ₘ'NR₇R₁₇, -ZC(Z)R₃ or -(CR₁₀R₂₀)ₘNR₁₃R₁₄;
- R₂: is C₁₋₁₀ alkyl N₃, -(CR₁₀R₂₀)ₙ'OR₉, heterocyclyl, heterocyclylC₁₋₁₀ alkyl, C₁₋₁₀alkyl, halo-substituted C₁₋₁₀ alkyl, C₂₋₁₀ alkenyl, C₂₋₁₀ alkynyl, C₃₋₇ cycloalkyl, C₃₋₇cycloalkylC₁₋₁₀ alkyl, C₅₋₇ cycloalkenyl, C₅₋₇cycloalkenyl-C₁₋₁₀-alkyl, aryl, arylC₁₋₁₀ alkyl, heteroaryl, heteroaryl-C₁₋₁₀-alkyl, (CR₁₀R₂₀)ₙOR₁₁, (CR₁₀R₂₀)ₙS(O)ₘR₁₈, (CR₁₀R₂₀)ₙNHS(O)₂R₁₈, (CR₁₀R₂₀)ₙNR₁₃R₁₄, (CR₁₀R₂₀)ₙNO₂, (CR₁₀R₂₀)ₙCN, (CR₁₀R₂₀)ₙ'SO₂R₁₈, (CR₁₀R₂₀)ₙS(O)ₘ'NR₁₃R₁₄, (CR₁₀R₂₀)ₙC(Z)R₁₁, (CR₁₀R₂₀)ₙOC(Z)R₁₁, (CR₁₀R₂₀)ₙC(Z)OR₁₁, (CR₁₀R₂₀)ₙC(Z)NR₁₃R₁₄, (CR₁₀R₂₀)ₙC(Z)NR₁₁OR₉, (CR₁₀R₂₀)ₙNR₁₀C(Z)R₁₁, (CR₁₀R₂₀)ₙNR₁₀C(Z)NR₁₃R₁₄, (CR₁₀R₂₀)ₙN(OR₆)C(Z)NR₁₃R₁₄, (CR₁₀R₂₀)ₙN(OR₆)C(Z)R₁₁, (CR₁₀R₂₀)ₙC(=NOR₆)R₁₁, (CR₁₀R₂₀)ₙNR₁₀C(=NR₁₉)NR₁₃R₁₄, (CR₁₀R₂₀)ₙOC(Z)NR₁₃R₁₄, (CR₁₀R₂₀)ₙNR₁₀C(Z)NR₁₃R₁₄, (CR₁₀R₂₀)ₙNR₁₀C(Z)OR₁₀, 5-(R₁₈)-1,2,4-oxadizaol-3-yl or 4-(R₁₂)-5-(R₁₈R₁₉)-4,5-dihydro-1,2,4-oxadiazol-3-yl; wherein the aryl, arylalkyl, heteroaryl, heteroaryl alkyl, heterocyclic and heterocyclic alkyl groups may be optionally substituted;
- n: is an integer having a value of 1 to 10;
- n': is 0, or an integer having a value of 1 to 10;
- m: is 0, or the integer 1 or 2;
- Z: is oxygen or sulfur;
- m': is 1 or 2,
- R₃: is heterocyclyl, heterocyclylC₁₋₁₀ alkyl or R₈;
- R₅: is hydrogen, C₁₋₄ alkyl, C₂₋₄ alkenyl, C₂₋₄ alkynyl or NR₇R₁₇, excluding the moeities -SR₅ being -SNR₇R₁₇ and -SOR₅ being -SOH;
- R₆: is hydrogen, a pharmaceutically acceptable cation, C₁₋₁₀ alkyl, C₃₋₇ cycloalkyl, aryl, arylC₁₋₄ alkyl, heteroaryl, heteroarylalkyl, heterocyclyl, aroyl, or C₁₋₁₀ alkanoyl;
- R₇ and R₁₇: is each independently selected from hydrogen or C₁₋₄ alkyl or R₇ and R₁₇ together with the nitrogen to which they are attached form a heterocyclic ring of 5 to 7 members which ring optionally contains an additional heteroatom selected from oxygen, sulfur or NR₁₅;
- R₈: is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, arylC₁₋₁₀ alkyl, heteroaryl, heteroarylC₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, (CR₁₀R₂₀)ₙNR₁₃R₁₄; wherein the aryl, arylalkyl, heteroaryl, heteroaryl alkyl may be optionally substituted;
- R₉: is hydrogen, -C(Z)R₁₁ or optionally substituted C₁₋₁₀ alkyl, S(O)₂R₁₈, optionally substituted aryl or optionally substituted aryl-C₁₋₄ alkyl;
- R₁₀ and R₂₀: is each independently selected from hydrogen or C₁₋₄ alkyl;
- R₁₁: is hydrogen, C₁₋₁₀ alkyl, C₃₋₇ cycloalkyl, heterocyclyl, heterocyclyl C₁₋₁₀alkyl, aryl, arylC₁₋₁₀ alkyl, heteroaryl or heteroarylC₁₋₁₀ alkyl;
- R₁₂: is hydrogen or R₁₆;
- R₁₃ and R₁₄: is each independently selected from hydrogen or optionally substituted C₁₋₄ alkyl, optionally substituted aryl or optionally substituted aryl-C₁₋₄ alkyl, or together with the nitrogen which they are attached form a heterocyclic ring of 5 to 7 members which ring optionally contains an additional heteroatom selected from oxygen, sulfur or NR₉ ;
- R₁₅: is R₁₀ or C(Z)-C₁₋₄ alkyl;
- R₁₆: is C₁₋₄ alkyl, halo-substituted-C₁₋₄ alkyl, or C₃₋₇ cycloalkyl;
- R₁₈: is C₁₋₁₀ alkyl, C₃₋₇ cycloalkyl, heterocyclyl, aryl, arylalkyl, heterocyclyl, heterocyclyl-C₁₋₁₀alkyl, heteroaryl or heteroarylalkyl;
- R₁₉: is hydrogen, cyano, C₁₋₄ alkyl, C₃₋₇ cycloalkyl or aryl;
or a pharmaceutically acceptable salt thereof.

### DETAILED DESCRIPTION OF THE INVENTION

The novel compounds of Formula (I) may also be used in association with the veterinary treatment of mammals, other than humans, in need of inhibition of cytokine inhibition or production. In particular, cytokine mediated diseases for treatment, therapeutically or prophylactically, in animals include disease states such as those noted herein in the Methods of Treatment section, but in particular viral infections. Examples of such viruses include, but are not limited to, lentivirus infections such as, equine infectious anaemia virus, caprine arthritis virus, visna virus, or maedi virus or retrovirus infections, such as but not limited to feline immunodeficiency virus (FIV), bovine immunodeficiency virus, or canine immunodeficiency virus or other retroviral infections.

In Formula (I), suitable R₁ moieties includes 4-pyridyl, 4-pyrimidinyl, 4-quinolyl, 6-isoquinolinyl, 4-quinazolinyl, 1-imidazolyl and 1-benzimidazolyl, of which the 4-pyridyl, 4-pyrimidinyl and 4-quinolyl are preferred. More preferred is an optionally substituted 4-pyrimidinyl and an optionally substituted 4-pyridyl, and most preferred is the an optionally substituted 4-pyrimidinyl ring.

Suitable substituents for the R₁ heteroaryl rings are C₁₋₄ alkyl, halo, OH, C₁₋₄ alkoxy, C₁₋₄ alkylthio, C₁₋₄ alkylsulfinyl, CH₂OR₁₂, NR₁₀R₂₀, or an N-heterocyclyl ring which ring has from 5 to 7 members and optionally contains an additional heteroatom selected from oxygen, sulfur or NR₁₅. A preferred substituent for all R₁ moieties is C₁₋₄ alkyl, in particular methyl, and NR₁₀R₂₀, preferably where R₁₀ and R₂₀ are hydrogen or methyl, more preferably R₁₀ and R₂₀ are hydrogen. A more preferred substituent is the NR₁₀R₂₀ moiety. Preferred ring placement of the R₁ substituent for on the 4-pyridyl derivative is the 2-position, such as 2-methyl-4-pyridyl. Preferred ring placement on the 4-pyrimidinyl is also at the 2-position, such as 2-methyl-pyrimidine or 2-amino pyrimidine.

Suitably, R₄ is phenyl, naphth-1-yl or naphth-2-yl, or a heteroaryl, which is optionally substituted by one or two substituents. More preferably R₄ is a phenyl or naphthyl ring. Suitable substitutions for R₄ when this is a 4-phenyl, 4-naphth-1-yl, 5-naphth-2-yl or 6-naphth-2-yl moiety are one or two substituents each of which are independently selected from halogen, -SR₅, -SOR₅, -OR₆, CF₃, or -(CR₁₀R₂₀)ₘNR₁₀R₂₀, and for other positions of substitution on these rings preferred substitution is halogen, -S(O)ₘR₃, -OR₃, CF₃, -(CR₁₀R₂₀)ₘNR₁₃R₁₄, -NR₁₀C(Z)R₃ and -NR₁₀S(O)ₘR₃. Preferred substituents for the 4-position in phenyl and naphth-1-yl and on the 5-position in naphth-2-yl include halogen, especially fluoro and chloro and -SR₅ and -SOR₅ wherein R₅ is preferably a C₁₋₂ alkyl, more preferably methyl; of which the fluoro and chloro is more preferred, and most especially preferred is fluoro. Preferred substituents for the 3-position in phenyl and naphth-1-yl rings include: halogen, especially fluoro and chloro; -OR₃, especially C₁₋₄ alkoxy; CF₃, NR₁₀R₂₀, such as amino; -NR₁₀C(Z)R₃, especially -NHCO(C₁₋₁₀ alkyl); -NR₁₀S(O)ₘR₈, especially -NHSO₂(C₁₋₁₀ alkyl), and -SR₃ and -SOR₃ wherein R₃ is preferably a C₁₋₂ alkyl, more preferably methyl. When the phenyl ring is disubstituted preferably it is two independent halogen moieties, such as fluoro and chloro, preferably di-chloro and more preferably in the 3,4-position.

Preferably, the R₄ moiety is an unsubstituted or substituted phenyl moiety. More preferably, R₄ is phenyl or phenyl substituted at the 4-position with fluoro and/or substituted at the 3-position with fluoro, chloro, C₁₋₄ alkoxy, methane-sulfonamido or acetamido, or R₄ is phenyl di-substituted at the 3,4-position with chloro or fluoro, more preferably chloro. Most preferably R₄ is a 4-fluorophenyl.

In Formula (I), suitably Z is oxygen.

Suitably, R₂ is C₁₋₁₀ alkyl N₃, -(CR₁₀R₂₀)ₙ'OR₉, heterocyclyl, heterocyclylC₁₋₁₀ alkyl, C₁₋₁₀alkyl, halo-substituted C₁₋₁₀ alkyl, C₂₋₁₀ alkenyl, C₂₋₁₀ alkynyl, C₃₋₇ cycloalkyl, C₃₋₇cycloalkylC₁₋₁₀ alkyl, C₅₋₇ cycloalkenyl, C₅₋₇ cycloalkenyl C₁₋₁₀ alkyl, aryl, arylC₁₋₁₀ alkyl, heteroaryl, heteroarylC₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, (CR₁₀R₂₀)ₙNR₁₃R₁₄, (CR₁₀R₂₀)ₙNO₂, (CR₁₀R₂₀)ₙCN, (CR₁₀R₂₀)ₙ'SO₂R₁₈, (CR₁₀R₂₀)ₙS(O)_{m'}NR₁₃R₁₄, (CR₁₀R₂₀)ₙC(Z)R₁₁, (CR₁₀R₂₀)ₙOC(Z)R₁₁, (CR₁₀R₂₀)ₙC(Z)OR₁₁, (CR₁₀R₂₀)ₙC(Z)NR₁₃R₁₄, (CR₁₀R₂₀)ₙC(Z)NR₁₁OR₉, (CR₁₀R₂₀)ₙNR₁₀C(Z)R₁₁, (CR₁₀R₂₀)ₙNR₁₀C(Z)NR₁₃R₁₄, (CR₁₀R₂₀)ₙN(OR₆)C(Z)NR₁₃R₁₄, (CR₁₀R₂₀)ₙN(OR₆)C(Z)R₁₁, (CR₁₀R₂₀)ₙC(=NOR₆)R₁₁, (CR₁₀R₂₀)ₙNR₁₀C(=NR₁₉)NR₁₃R₁₄, (CR₁₀R₂₀)ₙOC(Z)NR₁₃R₁₄, (CR₁₀R₂₀)ₙNR₁₀C(Z)NR₁₃R₁₄, (CR₁₀R₂₀)ₙNR₁₀C(Z)OR₁₀, 5-(R₁₈)-1,2,4-oxadizaol-3-yl or 4-(R₁₂)-5-(R₁₈R₁₉)-4,5-dihydro-1,2,4-oxadiazol-3-yl;wherein the aryl, arylalkyl, heteroaryl, heteroarylalkyl, heterocyclic, and heterocyclicaklyl moieties may be optionally substituted; wherein n is an integer having a value of 1 to 10, m is 0, or the integer 1 or 2; n' is 0, or an integer having a value of 1 to 10; and m' is 1 or 2. Preferably n is 1 to 4.

Preferably R₂ is an optionally substituted heterocyclyl ring, and optionally substituted heterocyclylC₁₋₁₀ alkyl, an optionally substituted C₁₋₁₀ alkyl, an optionally substituted C₃₋₇cycloalkyl, an optionally substituted C₃₋₇cycloalkyl C₁₋₁₀ alkyl, (CR₁₀R₂₀)ₙC(Z)OR₁₁ group, (CR₁₀R₂₀)ₙNR₁₃R₁₄, (CR₁₀R₂₀)ₙNHS(O)₂R₁₈, (CR₁₀R₂₀)ₙS(O)ₘR₁₈, an optionally substituted aryl; an optionally substituted arylC₁₋₁₀ alkyl, (CR₁₀R₂₀)ₙOR₁₁, (CR₁₀R₂₀)ₙC(Z)R₁₁, or (CR₁₀R₂₀)ₙC(=NOR₆)R₁₁ group.

More preferably R₂ is an optionally substituted heterocyclyl ring, and optionally substituted heterocyclylC₁₋₁₀ alkyl, optionally substituted aryl, (CR₁₀R₂₀)ₙNR₁₃R₁₄, or (CR₁₀R₂₀)ₙC(Z)OR₁₁ group.

When R₂ is an optionally substituted heterocyclyl the ring is preferably a morpholino, pyrrolidinyl, or a piperidinyl group. When the ring is optionally substituted the substituents may be directly attached to the free nitrogen, such as in the piperidinyl group or pyrrole ring, or on the ring itself. Preferably the ring is a piperidine or pyrrole, more preferably piperidine. The heterocyclyl ring may be optionally substituted one to four times independently by halogen; C₁₋₄ alkyl; aryl, such as phenyl; aryl alkyl, such as benzyl - wherein the aryl or aryl alkyl moieties themselves may be optionally substitued (as in the definition section below); C(O)OR₁₁, such as the C(O)C₁₋₄ alkyl or C(O)OH moieities; C(O)H; C(O)C₁₋₄ alkyl, hydroxy substituted C₁₋₄ alkyl, C₁₋₄ alkoxy, S(O)ₘC₁₋₄ alkyl (wherein m is 0, 1, or 2), NR₁₀R₂₀ (wherein R₁₀ and R₂₀ are independently hydrogen or C₁₋₄alkyl).

Preferably if the ring is a piperidine, the ring is attached to the imidazole at the 4-position, and the substituents are directly on the available nitrogen, i.e. a 1-Formyl-4-piperidine, 1-benzyl-4-piperidine, 1-methyl-4-piperidine, 1-ethoxycarbonyl-4-piperidine. If the ring is substituted by an alkyl group and the ring is attached in the 4-position, it is preferably substituted in the 2 or 6 position or both, such as 2,2,6,6-tetramethyl-4-piperidine. Simiarly, if the ring is a pyrrole, the ring is attached to the imidazole at the 3-position, and the substituents are aldo directly on the available nitrogen.

When R₂ is an optionally substituted heterocyclyl C₁₋₁₀ alkyl group, the ring is preferably a morpholino, pyrrolidinyl, or a piperidinyl group. Preferably this alkyl moiety is from 1 to 4, more preferably 3 or 4, and most preferably 3, such as in a propyl group. Preferred heterocyclic alkyl groups include but are not limited to, morpholino ethyl, morpholino propyl, pyrrollidinyl propyl, and piperidinyl propyl moieties. The heterocyclic ring herein is also optionally substiuted in a similar manner to that indicated above for the direct attachement of the heterocycyl.

When R₂ is an optionally substituted C₃₋₇cycloalkyl, or an optionally substituted C₃₋₇cycloalkyl C₁₋₁₀ alkyl, the cycloalkyl group is preferably a C5 to C6 ring which ring may be optionally substituted 1 or more times independtly by halogen, such as fluorine, chlorine, bromine or iodine; hydroxy; C₁₋₁₀ alkoxy, such as methoxy or ethoxy; S(O)ₘ alkyl, wherein m is 0, 1, or 2, such as methyl thio, methylsulfinyl or methyl sulfonyl; amino, mono & di-substituted amino, such as in the NR₇R₁₇ group; or where the R₇R₁₇ may cyclize together with the nitrogen to which they are attached to form a 5 to 7 membered ring which optionally includes an additional heteroatom selected from O/N/S; C₁₋₁₀ alkyl, such as methyl, ethyl, propyl, isopropyl, or t-butyl; halosubstitued alkyl, such as CF₃; hydroxy substituted C₁₋₁₀alkyl; C(O)OR₁₁, such as the free acid or methyl ester derivative; an optionally substituted aryl, such as phenyl; an optionally substituted arylalkyl, such as benzyl of phenethyl; and further where these aryl moieties may also be substituted one to two times by halogen; hydroxy; C₁₋₁₀ alkoxy; S(O)ₘ alkyl; amino, mono & di-substituted amino, such as in the NR₇R₁₇ group; alkyl or halosubstitued alkyl.

When R₂ is (CR₁₀R₂₀)ₙNR₁₃R₁₄, R₁₃ and R₁₄ are as defined in Formula (I), that is R₁₃ and R₁₄ are each independently selected from hydrogen, optionally substituted C₁₋₄ alkyl, optionally substituted aryl or an optionally substituted aryl-C₁₋₄ alkyl, or together with the nitrogen which they are attached form a heterocyclic ring of 5 to 7 members which ring optionally contains an additional heteroatom selected from oxygen, sulfur or NR₉. It is recognized that in some instances this can yield the same moiety as a heterocyclic C₁₋₁₀ alkyl moiety noted above which is also a suitable R₂ variable. Preferably R₁₃ and R₁₄ are independently hydrogen, C₁₋₄ alkyl, preferably methyl, or benzyl. The n term is preferably 1 to 4, more preferably 3 or 4, and most preferably 3, such as in a propyl group. Preferred groups include, but are not limited to, aminopropyl, (N-methyl-N-benzyl)aminopropyl, (N-Phenylmethyl)amino-1-propyl, or diethylamino propyl.

When R₂ is a (CR₁₀R₂₀)ₙC(Z)OR₁₁ group, R₁₁ is suitably hydrogen, C1-4 alkyl, especially methyl. The n term is preferably 1 to 4, more preferably 2 or 3, such as in an ethyl or propyl group. Preferred groups include, but are not limited to, carboxymethyl-1-butyl, carboxy-1-propyl, or 2-acetoxyethyl.

When R₂ is a (CR₁₀R₂₀)ₙS(O)ₘR₁₈ group m is 0, 1, or 2, and R₁₈ is preferably aryl, especially phenyl, or C₁₋₁₀ alkyl, especially methyl. The n term is preferably 1 to 4, more preferably 2 or 3, such as in an ethyl or propyl group.

When R₂ is a (CR₁₀R₂₀)ₙOR₁₁ group, R₁₁ is suitably hydrogen, aryl, especially phenyl, or C₁₋₁₀ alkyl, especially methyl or ethyl. The n term is preferably 1 to 4, more preferably 2 or 3, such as in an ethyl or propyl group.

When R₂ is a (CR₁₀R₂₀)ₙNHS(O)₂R₁₈ group, R₁₈ is suitably alkyl, especially methyl. The n term is preferably 1 to 4, more preferably 2 or 3, such as in an ethyl or propyl group.

When R₂ is a optionally substituted aryl, the aryl is preferably phenyl. The aryl ring may be optionally substituted one or more times, preferably by one or two substituents, independently selected from C₁₋₄ alkyl, halogen, especially fluoro or chloro, (CR₁₀R₂₀)ₜOR₁₁, -(CR₁₀R₂₀)ₜNR₁₀R₂₀, especially amino or mono- or di-alkylamino -(CR₁₀R₂₀)ₜS(O)ₘR₁₈, wherein m is 0, 1 or 2 ;- SH-, -(CR₁₀R₂₀)ₙNR₁₃R₁₄, -NR₁₀C(Z)R₃ (such -NHCO(C₁₋₁₀ alkyl)); -NR₁₀S(O)ₘR₈ (such as -NHSO₂(C₁₋₁₀ alkyl)); t is 0, or an integer of 1 to 4. Preferably the phenyl is substituted in the 3 or 4- position by -(CR₁₀R₂₀)ₜS(O)ₘR₁₈, and R₁₈ is preferably C₁₋₁₀ alkyl, especially methyl.

When R₂ is an optionally substituted heteroaryl or heteroarylalkyl group the ring may be optionally substituted one or more times, preferably by one or two substituents, independently selected from one or more times, by C₁₋₄ alkyl, halogen, especially fluoro or chloro, (CR₁₀R₂₀)ₜOR₁₁, -(CR₁₀R₂₀)ₜNR₁₀R₂₀, especially amino or mono- or di-alkylamino -(CR₁₀R₂₀)ₜS(O)ₘR₁₈, wherein m is 0, 1 or 2 ;-SH-, -(CR₁₀R₂₀)ₙ-NR₁₃R₁₄, -NR₁₀C(Z)R₃ (such -NHCO(C₁₋₁₀ alkyl)); -NR₁₀S(O)ₘR₈ (such as -NHSO₂(C₁₋₁₀ alkyl)); t is 0, or an integer of 1 to 4.

One skilled in the art would readily recognize that when R₂ is a (CR₁₀R₂₀)ₙOC(Z)R₁₁, or (CR₁₀R₂₀)ₙOC(Z)NR₁₃R₁₄ moiety, or any similarly substituted group that n is preferably at least 2 which will allow for the synthesis of stable compounds.

Preferably R₂ is a C₁₋₄ alkyl (branched and unbranched), especially methyl, a methylthio propyl, a methylsulfinyl propyl, an amino propyl, N-methyl-N-benzylamino propyl group, diethylamino propyl, cyclopropyl methyl, morpholinyl butyl, morpholinyl propyl, a morpholinyl ethyl, a piperidine or a substituted piperidine. More preferably R₂ is a methyl, isopropyl, butyl, t-butyl, n-propyl, methylthiopropyl, or methylsulfinyl propyl, morpholino propyl, morpholinyl butyl, phenyl substituted by halogen, thioalkyl or sulfinyl alkyl such as a methylthio, methylsulfinyl or methylsulfonyl moiety; piperidinyl, 1-Formyl-4-piperidine, 1-benzyl-4-piperidine, 1-methyl-4-piperidine, or a 1-ethoxycarbonyl-4-piperidine.

In all instances herein where there is an alkenyl or alkynyl moiety as a substituent group, the unsaturated linkage, i.e., the vinylene or acetylene linkage is preferably not directly attached to the nitrogen, oxygen or sulfur moieties, for instance in OR₃, or for certain R₂ moieties.

As used herein, "optionally substituted" unless specifically defined shall mean such groups as halogen, such as fluorine, chlorine, bromine or iodine; hydroxy; hydroxy substituted C₁₋₁₀alkyl; C₁₋₁₀ alkoxy, such as methoxy or ethoxy; S(O)m alkyl, wherein m is 0, 1 or 2, such as methyl thio, methylsulfinyl or methyl sulfonyl; amino, mono & di-substituted amino, such as in the NR₇R₁₇ group; or where the R₇R₁₇ may together with the nitrogen to which they are attached cyclize to form a 5 to 7 membered ring which optionally includes an additional heteroatom selected from O/N/S; C₁₋₁₀ alkyl, cycloalkyl, or cycloalkyl alkyl group, such as methyl, ethyl, propyl, isopropyl, t-butyl, etc. or cyclopropyl methyl; halosubstituted C₁₋₁₀ alkyl, such CF₃; an optionally substituted aryl, such as phenyl, or an optionally substituted arylalkyl, such as benzyl or phenethyl, wherein these aryl moieties may also be substituted one to two times by halogen; hydroxy; hydroxy substituted alkyl; C₁₋₁₀ alkoxy; S(O)ₘ alkyl; amino, mono & di-substituted amino, such as in the NR₇R₁₇ group; alkyl, or CF₃.

In a preferred subgenus of compounds of Formula (I), R₁ is 4-pyridyl, 2-alkyl-4-pyridyl, 4-quinolyl, 4-pyrimidinyl, or 2-amino-4-pyrimidinyl; R₂ is morpholinyl propyl, aminopropyl, piperidinyl, N-benzyl-4-piperidine, or a N-methyl-4-piperidine; and R₄ is phenyl or phenyl substituted one or two times by fluoro, chloro, C₁₋₄ alkoxy, -S(O)ₘ alkyl, methanesulfonamido or acetamido.

A preferred subgrouping of compounds of Formula (I) are those where R₂ is other than methyl when R₁ is pyridyl, and R₄ is an optionally substituted phenyl.

Suitable pharmaceutically acceptable salts are well known to those skilled in the art and include basic salts of inorganic and organic acids, such as hydrochloric acid, hydrobromic acid, sulphuric acid, phosphoric acid, methane sulphonic acid, ethane sulphonic acid, acetic acid, malic acid, tartaric acid, citric acid, lactic acid, oxalic acid, succinic acid, fumaric acid, maleic acid, benzoic acid, salicylic acid, phenylacetic acid and mandelic acid. In addition, pharmaceutically acceptable salts of compounds of Formula (I) may also be formed with a pharmaceutically acceptable cation, for instance, if a substituent group comprises a carboxy moiety. Suitable pharmaceutically acceptable cations are well known to those skilled in the art and include alkaline, alkaline earth, ammonium and quaternary ammonium cations.

The following terms, as used herein, refer to:
- "halo" or "halogens", include the halogens: chloro, fluoro, bromo and iodo.
- "C₁₋₁₀alkyl" or "alkyl" - both straight and branched chain radicals of 1 to 10 carbon atoms, unless the chain length is otherwise limited, including, but not limited to, methyl, ethyl, *n*-propyl, *iso*-propyl, *n*-butyl, *sec*-butyl, *iso*-butyl, *tert*-butyl, *n*-pentyl and the like.
- The term "cycloalkyl" is used herein to mean cyclic radicals, preferably of 3 to 8 carbons, including but not limited to cyclopropyl, cyclopentyl, cyclohexyl, and the like.
- The term "cycloalkenyl" is used herein to mean cyclic radicals, preferably of 5 to 8 carbons, which have at least one bond including but not limited to cyclopentenyl, cyclohexenyl, and the like.
- The term "alkenyl" is used herein at all occurrences to mean straight or branched chain radical of 2-10 carbon atoms, unless the chain length is limited thereto, including, but not limited to ethenyl, 1-propenyl, 2-propenyl, 2-methyl-1-propenyl, 1-butenyl, 2-butenyl and the like.
- "aryl" - phenyl and naphthyl;
- "heteroaryl" (on its own or in any combination, such as "heteroaryloxy", or "heteroaryl alkyl") - a 5-10 membered aromatic ring system in which one or more rings contain one or more heteroatoms selected from the group consisting of N, O or S, such as, but not limited, to pyrrole, pyrazole, furan, thiophene, quinoline, isoquinoline, quinazolinyl, pyridine, pyrimidine, oxazole, thiazole, thiadiazole, triazole, imidazole, or benzimidazole.
- "heterocyclic" (on its own or in any combination, such as "heterocyclylalkyl") - a saturated or partially unsaturated 4-10 membered ring system in which one or more rings contain one or more heteroatoms selected from the group consisting of N, O, or S; such as, but not limited to, pyrrolidine, piperidine, piperazine, morpholine, tetrahydro pyran, or imidazolidine.
- The term "aralkyl" or "heteroarylalkyl" or "heterocyclicalkyl" is used herein to mean C₁₋₄ alkyl as defined above attached to an aryl, heteroaryl or heterocyclic moiety as also defined herein unless otherwise indicate.
- "sulfinyl" - the oxide S (O) of the corresponding sulfide, the term "thio" refers to the sulfide, and the term "sulfonyl" refers to the fully oxidized S (O)₂ moiety.
- "aroyl" - a C(O)Ar, wherein Ar is as phenyl, naphthyl, or aryl alkyl derivative such as defined above, such group include but are note limited to benzyl and phenethyl.
- "alkanoyl" - a C(O)C₁₋₁₀ alkyl wherein the alkyl is as defined above.

The compounds of the present invention may contain one or more asymmetric carbon atoms and may exist in racemic and optically active forms. All of these compounds are included within the scope of the present invention.

Exemplified compounds of Formula (I) include:
1-[3-(4-Morpholinyl)propyl]-4-(4-fluorophenyl)-5-(4-pyridyl)imidazole;
1-(3-Chloropropyl)-4-(4-fluorophenyl)-5-(4-pyridyl)imidazole;
1-(3-Azidopropyl)-4-(4-fluorophenyl)-5-(4-pyridyl)imidazole; 1-(3-Aminopropyl)-4-(4-fluorophenyl)-5-(4-pyridyl)imidazole;
1-(3-Methylsulfonamidopropyl)-4-(4-fluorophenyl)-5-(4-pyridyl)imidazole;
1-[3-(N-Phenylmethyl)aminopropyl]-4-(4-fluorophenyl)-5-(4-pyridyl)imidazole;
1-[3-(N-Phenylmethyl-N-methyl)aminopropyl)-4-(4-fluorophenyl)-5-(4-pyridyl)imidazole;
1-[3-(1-Pyrrolidinyl)propyl]-4-(4-fluorophenyl)-5-(4-pyridyl)imidazole;
1-(3-Diethylaminopropyl)-4-(4-fluorophenyl)-5-(4-pyridyl)imidazole;
1-[3-(1-Piperidinyl)propyl]-4-(4-fluorophenyl)-5-(4-pyridyl)imidazole;
1-[3-(Methylthio)propyl]-4-(4-fluorophenyl)-5-(4-pyridyl)imidazole;
1-[2-(4-Morpholinyl)ethyl]-4-(4-fluorophenyl)-5-(4-pyridyl)imidazole;
1-[3-(4-Morpholinyl)propyl]-4-(3-methylthiophenyl)-5-(4-pyridyl)imidazole;
1-[3-(4-Morpholinyl)propyl]-4-(3-methylsulfinylphenyl)-5-(4-pyridyl)imidazole;
1-[3-(N-methyl-N-benzyl)aminopropyl]-4-(3-methylthiophenyl)-5-(4-pyridyl)imidazole;
1-[3-(N-methyl-N-benzyl)aminopropyl]-4-(3-methylsulfinylphenyl)-5-(4-pyridyl)imidazole;
1-[4-(Methylthio)phenyl]-4-(4-fluorophenyl)-5-(4-pyridyl)imidazole;
1-[4-(Methylsulfinyl)phenyl]-4-(4-fluorophenyl)-5-(4-pyridyl)imidazole;
1-[3-(Methylthio)phenyl]-4-(4-fluorophenyl)-5-(4-pyridyl)imidazole;
1-[3-(Methylsulfinyl)phenyl]-4-(4-fluorophenyl)-5-(4-pyridyl)imidazole;
1-[2-(Methylthio)phenyl]-4-(4-fluorophenyl)-5-(4-pyridyl)imidazole;
1-[2-(Methylsulfinyl)phenyl]-4-(4-fluorophenyl)-5-(4-pyridyl)imidazole;
1-[4-(4-Morpholinyl)butyl]-4-(4-fluorophenyl)-5-(4-pyridyl)imidazole;
1-Cyclopropyl-4-(4-fluorophenyl)-5-(4-pyridyl)imidazole;
1-Isopropyl-4-(4-fluorophenyl)-5-(4-pyridyl)imidazole; 1-Cyclopropylmethyl-4-(4-fluorophenyl)-5-(4-pyridyl)imidazole;
1-tert-Butyl-4-(4-fluorophenyl)-5-(4-pyridyl)imidazole;
1-(2,2-Diethoxyethyl)-4-(4-fluorophenyl)-5-(4-pyridyl)imidazole;
1-Formylmethyl-4-(4-fluorophenyl)-5-(4-pyridyl)imidazole;1-Hydroxyiminylmethyl-4-(4-fluorophenyl)-5-(4-pyridyl)imidazole; 1-Cyanomethyl-4-(4-fluorophenyl)-5-(4-pyridyl)imidazole;
1-[3-(4-Morpholinyl)propyl)-4-(4-fluorophenyl)-5-(2-methylpyrid-4-yl)imidazole;4-(4-Fluorophenyl)-1-[3-(4-morpholinyl)propyl]-5-(2-chloropyridin-4-yl) imidazole; 4-(4-Fluorophenyl)-1-[3-(4-morpholinyl)propyl]-5-(2-amino-4-pyridinyl) imidazole; 1-(4-Carboxymethyl)propyl-4-(4-fluorophenyl)-5-(4-pyridyl)imidazole;
1-(4-Carboxypropyl)-4-(4-fluorophenyl)-5-(4-pyridyl)imidazole;
1-(3-Carboxymethyl)ethyl-4-(4-fluorophenyl)-5-(4-pyridyl)imidazole;
1-(3-Carboxy)ethyl-4-(4-fluorophenyl)-5-(4-pyridyl)imidazole;
1-(1-Benzylpiperidin-4-yl)-4-(4-fluorophenyl)-5-(4-pyridyl) imidazole;
5-(2-Aminopyrimidin-4-yl)-4-(4-fluorophenyl)-1-[3-(4-Morpholinyl)propyl] imidazole;
5-(2-Aminopyrimidin-4-yl)-4-(4-fluorophenyl)-1-(1-benzylpiperidin-4-yl)imidazole; 5-(2-Aminopyrimidin-4-yl)-4-(4-fluorophenyl)-1-(2-propyl)imidazole; 5-(2-Aminopyrimidin-4-yl)-4-(4-fluorophenyl)-1-(cyclopropylmethyl)imidazole; 5-(2-Aminopyrimidin-4-yl)-4-(4-fluorophenyl)-1-(1-carboxyethyl-4-piperidinyl)imidazole;
5-(2-Aminopyrimidin-4-yl)-4-(4-fluorophenyl)-1-(4-piperidinyl) imidazole;
1-Methyl-4-phenyl-5-(4-pyridyl)imidazole;
1-Methyl-4-[3-(chlorophenyl)]-5-[4-pyridinyl]imidazole;
1-Methyl-4-(3-methylthiophenyl)-5-(4-pyridyl)imidazole;
1-Methyl-4-(3-methylsulfinylphenyl)-5-(4-pyridyl)imidazole; (+/-)-4-(4-Fluorophenyl)-1-[3-(methylsulfinyl)propyl]-5-(4-pyridinyl)imidazole;
4-(4-Fluorophenyl)-1-[(3-methylsulfonyl)propyl]-5-(4-pyridinyl)imidazole;
1-(3-Phenoxypropyl)-4-(4-fluorophenyl)-5-(4-pyridinyl)imidazole;
1-[3-(Phenylthio)propyl]-4-(4-fluorophenyl)-5-(4-pyridinyl)imidazole;
1-[3-(4-Morpholinyl)propyl]-4-(4-fluorophenyl)-5-(4-quinolyl)imidazole;
(+/-)-1-(3-Phenylsulfinylpropyl)-4-(4-fluorophenyl)-5-(4-pyridinyl)imidazole;
1-(3-Ethoxypropyl)-4-(4-fluorophenyl)-5-(4-pyridinyl)imidazole;
1-(3-Phenylsulfonylpropyl-4-(4-fluorophenyl)-5-(4-pyridinyl)imidazole;
1-[3-(4-Morpholinyl)propyl]-4-(3-chlorophenyl)-5-(4-pyridyl)imidazole;
1-[3-(4-Morpholinyl)propyl]-4-(3,4-dichlorophenyl)-5-(4-pyridyl)imidazole ;
4-[4-(4-Fluorophenyl)-1-[3-(4-morpholinyl)propyl]-5-(pyrimid-2-one-4-yl)imidazole;
4-(4-Fluorophenyl)-5-[2-(methylthio)-4-pyrimidinyl]-1-[3-(4-morpholinyl)propyl]-imidazole; 4-(4-Fluorophenyl)-5-[2-(methylsulfinyl)-4-pyrimidinyl]-1-[3-(4-morpholinyl)- propyl]imidazole; (E)-1-(1-Propenyl)-4-(4-fluorophenyl)-5-(4-pyridinyl)imidazole;
1-(2-Propenyl)-4-(4-fluorophenyl)-5-(4-pyridinyl)imidazole;
5-[(2-N,N-Dimethylamino)pyrimidin-4-yl]-4-(4-fluorophenyl)-1-[3-(4-morpholinyl)-propyl]imidazole; 1-[3-(4-Morpholinyl)propyl]-5-(4-pyridinyl)-4-[4-(trifluoromethyl)phenyl]imidazole;1-[3-(4-Morpholinyl)propyl]-5-(4-pyridinyl)-4-[3-(trifluoromethyl)phenyl]imidazole ;
1-(Cyclopropylmethyl)-4-(3,4-dichlorophenyl)-5-(4-pyridinyl)imidazole; 1-(Cyclopropylmethyl)-4-(3-trifluoromethylphenyl)-5-(4-pyridinyl)imidazole;
1-(Cyclopropylmethyl)-4-(4-fluorophenyl)-5-(2-methylpyrid-4-yl)imidazole;
1-[3-(4-Morpholinyl)propyl]-5-(4-pyridinyl)-4-(3,5-bistrifluoromethylphenyl)-imidazole
5-[4-(2-Aminopyrimidinyl)]-4-(4-fluorophenyl)-1-(2-carboxy-2,2-dimethylethyl)imidazole; 1-(1-Formyl-4-piperidinyl)-4-(4-fluorophenyl)-5-(4-pyridinyl)imidazole;
5-(2-Amino-4-pyrimidinyl)-4-(4-fluorophenyl)-1-(1-methyl-4-piperidinyl)imidazole;
1-(2,2-Dimethyl-3-morpholin-4-yl)propyl-4-(4-fluorophenyl)-5-(2-Amino-4-pyrimidinyl)imidazole;
4-(4-Fluorophenyl)-5-(4-pyridyl)-1-(2-acetoxyethyl)imidazole, 5-(2-Aminopyrimidin-4-yl)-4-(4-fluorophenyl)-1-(1-benzylpyrrolin-3-yl)imidazole; 5-(2-Aminopyrimidin-4-yl)-4-(4-fluorophenyl)-1-(2,2,6,6-tetramethylpiperidin-4- yl)imidazole.

Preferred compounds of Formula (I) include:
5-[4-(2-Amino)pyrimidinyl]-4-(4-fluorophenyl)-1-(4-N-morpholino-1-propyl)imidazole;
5-[4-(2-Aminopyrimidinyl)-4-(4-fluorophenyl)-1-(1-benzyl-4-piperidinyl)imidazole; 5-(2-Amino-4-pyrimidinyl)-4-(4-fluorophenyl)-1-(4-piperidinyl)imidazole;
5-(2-Amino-4-pyrimidinyl)-4-(4-fluorophenyl)-1-(1-methyl-4-piperidinyl)-imidazole.

Another aspect of the present invention is the compound, 4-phenyl-5-[4-pyridyl)imidazole. Another aspect is of the present invention is a pharmaceutical composition comprising a carrier or diluent and effective amount of 4-phenyl-5-[4-pyridyl)imidazole. Yet another aspect of the present invention is the novel method of treating a cytokine mediated disease state, in a mammal in need thereof, with an effective amount of 4-phenyl-5-[4-pyridyl)imidazole.

For purposes herein the dosage ranges, formulation details, and methods of making are analogous to the compounds of Formula (I).

The compounds of Formula (I) may be obtained by applying synthetic procedures, some of which are illustrated in Schemes I and V. The synthesis provided for in these Schemes is applicable for the producing compounds of Formula (I) having a variety of different R₁, R₂, and R₄ groups which are reacted, employing optional substituents which are suitably protected, to achieve compatibility with the reactions outlined herein. Subsequent deprotection, in those cases, then affords compounds of the nature generally disclosed. Once the imidazole nucleus has been established, further compounds of Formula (I) may be prepared by applying standard techniques for functional group interconversion, well known in the art.

For instance: -C(O)NR₁₃R₁₄ from -CO₂CH₃ by heating with or without catalytic metal cyanide, e.g. NaCN, and HNR₁₃R₁₄ in CH₃OH; -OC(O)R₃ from -OH with e.g., ClC(O)R₃ in pyridine; -NR₁₀-C(S)NR₁₃R₁₄ from -NHR₁₀ with an alkylisothiocyante or thiocyanic acid; NR₆C(O)OR₆ from -NHR₆ with the alkyl chloroformate; -NR₁₀C(O)NR₁₃R₁₄ from -NHR₁₀ by treatment with an isocyanate, e.g. HN=C=O or R₁₀N=C=O; -NR₁₀-C(O)R₈ from -NHR₁₀ by treatment with Cl-C(O)R₃ in pyridine; -C(=NR₁₀)NR₁₃R₁₄ from -C(NR₁₃R₁₄)SR₃ with H₃NR₃⁺OAc⁻ by heating in alcohol; -C(NR₁₃R₁₄)SR₃ from -C(S)NR₁₃R₁₄ with R₆-I in an inert solvent, e.g. acetone; -C(S)NR₁₃R₁₄ (where R₁₃ or R₁₄ is not hydrogen) from -C(S)NH₂ with HNR₁₃R₁₄-C(=NCN)-NR₁₃R₁₄ from -C(=NR₁₃R₁₄)-SR₃ with NH₂CN by heating in anhydrous alcohol, alternatively from -C(=NH)-NR₁₃R₁₄ by treatment with BrCN and NaOEt in EtOH; -NR₁₀-C(=NCN)SR₈ from -NHR₁₀ by treatment with (R₈S)₂C=NCN; -NR₁₀SO₂R₃ from -NHR₁₀ by treatment with ClSO₂R₃ by heating in pyridine; -NR₁₀C(S)R₃ from -NR₁₀C(O)R₈ by treatment with Lawesson's reagent [2,4-*bis*(4-methoxyphenyl)-1,3,2,4-dithiadiphosphetane-2,4-disulfide]; -NR₁₀SO₂CF₃ from -NHR₆ with triflic anhydride and base wherein R₃, R₆, R₁₀, R₁₃ and R₁₄ are as defined in Formula (I) herein.

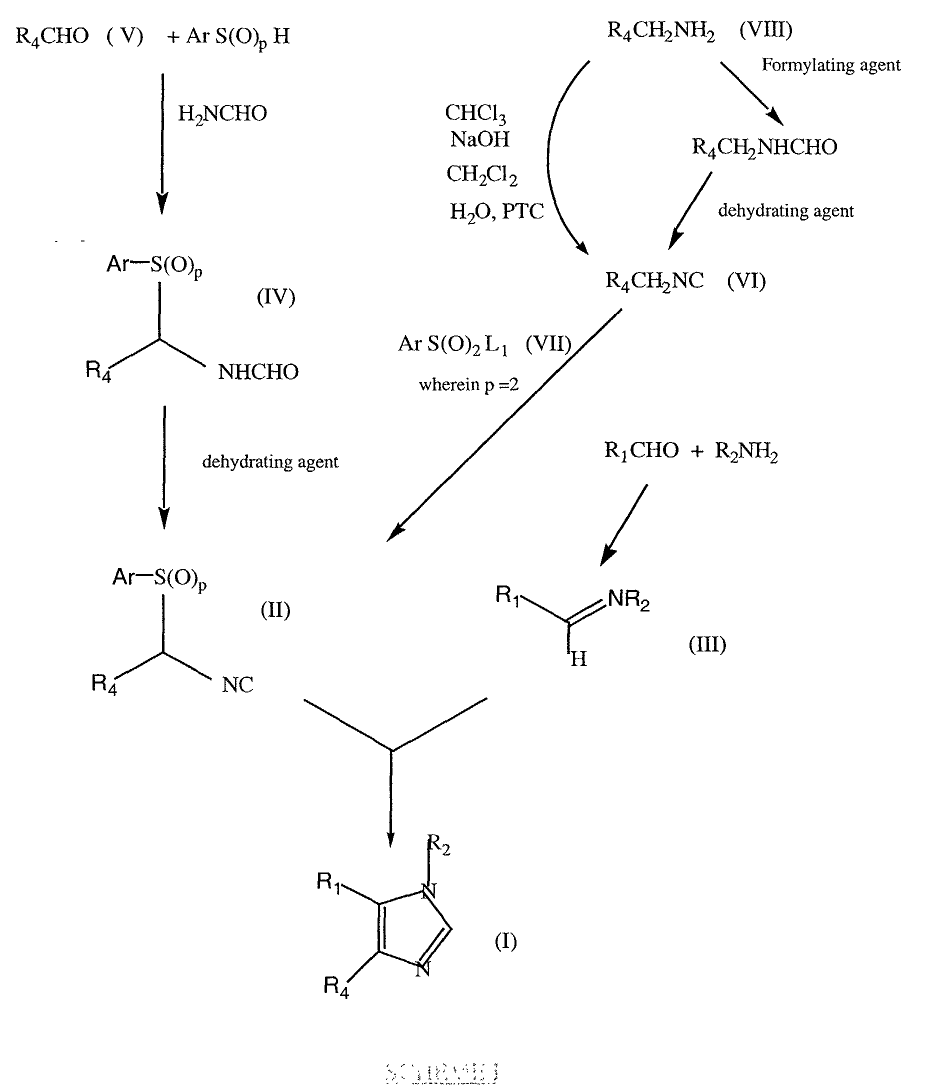

Referring to Scheme I the compounds of Formula (I) are suitably prepared by reacting a compound of the Formula (II) with a compound of the Formula (III) wherein p is 0, 1 or 2, R₁, R₂ and R₄ are as defined herein, or are precursors of the groups R₁, R₂ and R₄, and Ar is an optionally substituted phenyl group, and thereafter if necessary converting a precursor of R₁, R₂ and R₄ to a group R₁, R₂ and R₄.

Suitably Ar is phenyl optionally substituted by C₁₋₄alkyl, C₁₋₄ alkoxy or halo. Preferably Ar is phenyl or 4-methylphenyl. Suitably the reaction is performed at ambient temperature or with cooling (e.g. -50° to 10° ) in an inert solvent such as methylene chloride, tetrahydrofuran, toluene or dimethoxyethane in the presence of an appropriate base such as 1,8-diazabicyclo [5.4.0.] undec-7-ene (DBU) or a guanidine base such as 1,5,7- triazabicyclo [4.4.0] dec-5-ene (TBD). Suitably p is 0 or 2, preferably p is 0 in which case the intermediates of formula (II) are very stable and capable of storage for a long time.

Precursors of the groups R₁, R₂ and R₄ can be other R₁, R₂ and R₄ groups which can be interconverted by applying standard techniques for functional group interconversion. For example a compound of the formula (1) wherein R₂ is halo - substituted C₁₋₁₀ alkyl can be converted to the corresponding C₁₋₁₀ alkylN₃ derivative by reacting with a suitable azide salt, and thereafter if desired can be reduced to the corresponding C₁₋₁₀alkylNH₂ compound, which in turn can be reacted with R₁₈S(0)₂X wherein X is halo (e.g., chloro) to yield the corresponding C₁₋₁₀alkylNHS(0)₂R₁₈ compound.

Alternatively a compound of the formula (1) where R₂ is halo-substituted C₁₋₁₀-alkyl can be reacted with an amine R₁₃R₁₄NH to yield the corresponding C₁₋₁₀-alkylNR₁₃R₁₄ compound, or can be reacted with an alkali metal salt of R₁₈SH to yield the corresponding C₁₋₁₀alkylSR₁₈ compound.

In a further aspect the present invention provides compounds of the Formula (II) as defined herein with the proviso that when ArS(0)ₚ is tosyl, then R₄ is not an unsubstituted phenyl.

The compounds of Formula (II), in Scheme I, may be prepared by the methods of Van Leusen et al., JOC, 42, 1153 (1977). For example a compound of the Formula (II) may be prepared by dehydrating a compound of the Formula (IV) wherein Ar, R₄ and p are as defined herein.

Suitable dehydrating agents include phosphorus oxychloride, oxalyl chloride or tosyl chloride in the presence of a suitable base such as triethylamine.

The compounds of formula (IV) may be prepared by reacting a compound of the formula (V) R₄CHO where R₄ is as defined herein, with ArS(0)ₚH and formamide under dehydrating conditions at ambient or elevated temperature e.g. 30° to 150°, conveniently at reflux, optionally in the presence of an acid catalyst. Alternatively trimethysilylchloride can be used in place of the acid catalyst. Examples of acid catalysts include camphor-10-sulphonic acid, p-toluenesulphonic acid, hydrogen chloride or sulphuric acid.

The compounds of the formula (II) where p is 2 may also be prepared by reacting in the presence of a strong base a compound of the formula (VI) R₄CH₂NC with a compound of the formula (VII) ArSO₂L₁ wherein R₄ and Ar are as defined herein and L₁ is a leaving group such as halo, e.g. fluoro. Suitable strong bases include alkyl lithiums such as butyl lithium or lithium diisopropylamide.

The compounds of formula (VI) may be prepared by reacting a compound of the folmula (VIII) R₄CH₂NH₂ with an alkyl formate (e.g. ethylformate) to yield an intermediate amide which can be converted to the desired isonitrile by reacting with a dehydrating agent such as oxalylchloride, phosphorus oxychloride or tosyl chloride in the presence of a suitable base such as triethylamine.

Alternatively a compound of the formula (VIII) may be converted to a compound of the formula (VI) by reaction with chloroform and sodium hydroxide in aqueous dichloromethane under phase transfer catalysis.

The compounds of the formula (III) may be prepared by reacting a compound of the formula R₁CHO with a primary amine R₂NH₂.

The amino compounds of the formula (VIII) are known or can be prepared from the corresponding alcohols, oximes or amides using standard functional group interconversions.

In a further process, compounds of Formula (I) may be prepared by coupling a suitable derivative of a compound of Formula (IX): 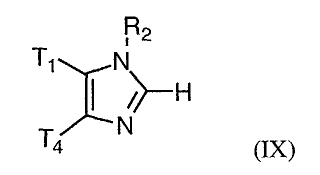 wherein T₁ is hydrogen and T₄ is R₄, or alternatively T₁ is R₁ and T₄ is H in which R₁, R₂ and R₄ are as hereinbefore defined; with: (i) when T₁ is hydrogen, a suitable derivative of the heteroaryl ring R₁H, under ring coupling conditions, to effect coupling of the heteroaryl ring R₁ to the imidazole nucleus at position 5; (ii) when T₄ is hydrogen, a suitable derivative of the aryl ring R₄H, under ring coupling conditions, to effect coupling of the aryl ring R₄ to the imidazole nucleus at position 4.

Such aryl/heteroaryl coupling reactions are well known to those skilled in the art. In general, an organometallic synthetic equivalent of an anion of one component is coupled with a reactive derivative of the second component, in the presence of a suitable catalyst. The anion equivalent may be formed from either the imidazole of Formula (IX), in which case the aryl/heteroaryl compound provides the reactive derivative, or the aryl/heteroaryl compound in which case the imidazole provides the reactive derivative. Accordingly, suitable derivatives of the compound of Formula (IX) or the aryl/heteroaryl rings include organometallic derivatives such as organomagnesium, organozinc, organostannane and boronic acid derivatives and suitable reactive derivatives include the bromo, iodo, fluorosulfonate and trifluoromethanesulphonate derivatives. Suitable procedures are described in WO 91/19497, the disclosure of which is incorporated by reference herein.

Suitable organomagnesium and organozinc derivatives of a compound of Formula (IX) may be reacted with a halogen, fluorosulfonate or triflate derivative of the heteroaryl or aryl ring, in the presence of a ring coupling catalyst, such as a palladium (O) or palladium (II) catalyst, following the procedure of Kumada *et a*l., Tetrahedron Letters, **22,** 5319 (1981). Suitable such catalysts include *tetrakis*(triphenylphosphine)palladium and PdCl₂[1,4-*bis*-(diphenylphosphino)-butane], optionally in the presence of lithium chloride and a base, such as triethylamine. In addition, a nickel (II) catalyst, such as Ni(II)Cl₂(1,2-biphenylphosphino)ethane, may also be used for coupling an aryl ring, following the procedure of Pridgen, J Org Chem, 1982, **47,** 4319. Suitable reaction solvents include hexamethylphosphor-amide. When the heteroaryl ring is 4-pyridyl, suitable derivatives include 4-bromo- and 4-iodo-pyridine and the fluorosulfonate and triflate esters of 4-hydroxy pyridine. Similarly, suitable derivatives for when the aryl ring is phenyl include the bromo, fluorosulfonate, triflate and, preferably, the iodo-derivatives. Suitable organomagnesium and organozinc derivatives may be obtained by treating a compound of Formula (IX) or the bromo derivative thereof with an alkyllithium compound to yield the corresponding lithium reagent by deprotonation or transmetallation, respectively. This lithium intermediate may then be treated with an excess of a magnesium halide or zinc halide to yield the corresponding organometallic reagent.

A trialkyltin derivative of the compound of Formula (IX) may be treated with a bromide, fluorosulfonate, triflate, or, preferably, iodide derivative of an aryl or heteroaryl ring compound, in an inert solvent such as tetrahydrofuran, preferably containing 10% hexamethylphosphoramide, in the presence of a suitable coupling catalyst, such as a palladium (0) catalyst, for instance *tetrakis*(triphenylphosphine)palladium, by the method described in by Stille, J Amer Chem Soc, 1987, **109,** 5478, US Patents 4,719,218 and 5,002,942, or by using a palladium (II) catalyst in the presence of lithium chloride optionally with an added base such as triethylamine, in an inert solvent such as dimethyl formamide. Trialkyltin derivatives may be conveniently obtained by metallation of the corresponding compound of Formula (IX) with a lithiating agent, such as *s*-butyl-lithium or *n*-butyllithium, in an ethereal solvent, such as tetrahydrofuran, or treatment of the bromo derivative of the corresponding compound of Formula (IX) with an alkyl lithium, followed, in each case, by treatment with a trialkyltin halide. Alternatively, the bromo- derivative of a compound of Formula (IX) may be treated with a suitable heteroaryl or aryl trialkyl tin compound in the presence of a catalyst such as *tetrakis*-(triphenyl-phosphine)-palladium, under conditions similar to those described above.

Boronic acid derivatives are also useful. Hence, a suitable derivative of a compound of Formula (IX), such as the bromo, iodo, triflate or fluorosulphonate derivative, may be reacted with a heteroaryl- or aryl-boronic acid, in the presence of a palladium catalyst such as *tetrakis*-(triphenylphosphine)-palladium or PdCl₂[1,4-*bis*(diphenyl-phosphino)-butane] in the presence of a base such as sodium bicarbonate, under reflux conditions, in a solvent such as dimethoxyethane (see Fischer and Haviniga, Rec. Trav. Chim. Pays Bas, **84,** 439, 1965, Snieckus, V., Tetrahedron Lett., **29,** 2135, 1988 and Terashimia, M., Chem. Pharm. Bull., **11,** 4755, 1985). Nonaqueous conditions, for instance, a solvent such as DMF, at a temperature of about 100°C, in the presence of a Pd(II) catalyst may also be employed (see Thompson W J *et al*, J Org Chem, **49**, 5237, 1984). Suitable boronic acid derivatives may be prepared by treating the magnesium or lithium derivative with a trialkylborate ester, such as triethyl, tri-*iso*-propyl or tributylborate, according to standard procedures.

In such coupling reactions, it will be readily appreciated that due regard must be exercised with respect to functional groups present in the compounds of Formula (IX). Thus, in general, amino and sulfur substituents should be non-oxidised or protected.

Compounds of Formula (IX) are imidazoles and may be obtained by any of the procedures herein before described for preparing compounds of Formula (I). In particular, an α-halo-ketone or other suitably activated ketones R₄COCH₂Hal (for compounds of Formula (IX) in which T₁ is hydrogen) or R₁COCH₂Hal (for compounds of Formula (IX) in which T₄ is hydrogen) may be reacted with an amidine of the formula R₂NH-C=NH, wherein R₂ is as defined in Formula (I), or a salt thereof, in an inert solvent such as a halogenated hydrocarbon solvent, for instance chloroform, at a moderately elevated temperature, and, if necessary, in the presence of a suitable condensation agent such as a base. The preparation of suitable a-halo-ketones is described in WO 91/19497. Suitable reactive esters include esters of strong organic acids such as a lower alkane sulphonic or aryl sulphonic acid, for instance, methane or *p*-toluene sulphonic acid. The amidine is preferably used as the salt, suitably the hydrochloride salt, which may then be converted into the free amidine *in situ*, by employing a two phase system in which the reactive ester is in an inert organic solvent such as chloroform, and the salt is in an aqueous phase to which a solution of an aqueous base is slowly added, in dimolar amount, with vigorous stirring. Suitable amidines may be obtained by standard methods, see for instance, Garigipati R, Tetrahedron Letters, 190, **31**, 1989.

Compounds of Formula (I) may also be prepared by a process which comprises reacting a compound of Formula (IX), wherein T₁ is hydrogen, with an **N**-acyl heteroaryl salt, according to the method disclosed in US patent 4,803,279, US patent 4,719,218 and US patent 5,002,942, to give an intermediate in which the heteroaryl ring is attached to the imidazole nucleus and is present as a 1,4-dihydro derivative thereof, which intermediate may then be subjected to oxidative-deacylation conditions (Scheme II). The heteroaryl salt, for instance a pyridinium salt, may be either preformed or, more preferably, prepared *in situ* by adding a substituted carbonyl halide (such as an acyl halide, an aroyl halide, an arylalkyl haloformate ester, or, preferably, an alkyl haloformate ester, such as acetyl bromide, benzoylchloride, benzyl chloroformate, or, preferably, ethyl chloroformate) to a solution of the compound of Formula (IX) in the heteroaryl compound R₁H or in an inert solvent such as methylene chloride to which the heteroaryl compound has been added. Suitable deacylating and oxidising conditions are described in U.S. Patent Nos. 4,803,279, 4,719,218 and 5,002,942, which references are hereby incorporated by reference in their entirety. Suitable oxidizing systems include sulfur in an inert solvent or solvent mixture, such as decalin, decalin and diglyme, *p*-cymene, xylene or mesitylene, under reflux conditions, or, preferably, potassium *t*-butoxide in *t*-butanol with dry air or oxygen.

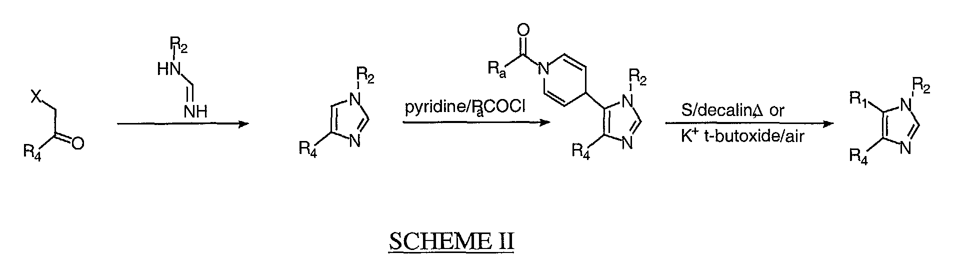

In a further process, illustrated in Scheme III below, compounds of Formula (I) may be prepared by treating a compound of Formula (X) thermally or with the aid of a cyclising agent such as phosphorus oxychloride or phosphorus pentachloride (see Engel and Steglich, Liebigs Ann Chem, 1978, 1916 and Strzybny *et al*., J Org Chem, 1963, **28,** 3381). Compounds of Formula (X) may be obtained, for instance, by acylating the corresponding a-keto-amine with an activated formate derivative such as the corresponding anhydride, under standard acylating conditions followed by formation of the imine with R₂NH₂. The aminoketone may be derived from the parent ketone by oxamination and reduction and the requisite ketone may in turn be prepared by decarboxylation of the beta-ketoester obtained from the condensation of an aryl (heteroaryl) acetic ester with the R₁COX component.

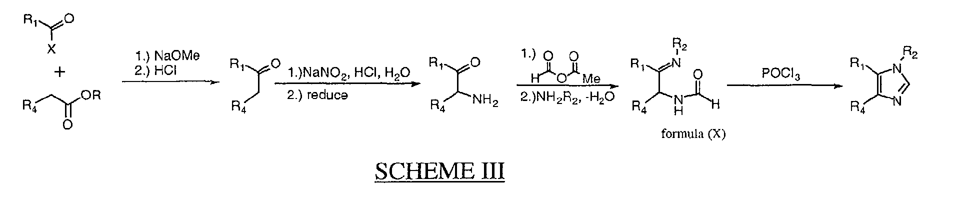

In Scheme IV illustrated below, two (2) different routes which use ketone (formula XI) for preparing a compound of Formula (I). A heterocyclic ketone (XI) is prepared by adding the anion of the alkyl heterocycle such as 4-methyl-quinoline (prepared by treatment thereof with an alkyl lithium, such as *n*-butyl lithium) to an **N**-alkyl-**O**-alkoxybenzamide, ester, or any other suitably activated derivative of the same oxidation state. Alternatively, the anion may be condensed with a benzaldehyde, to give an alcohol which is then oxidised to the ketone (XI).

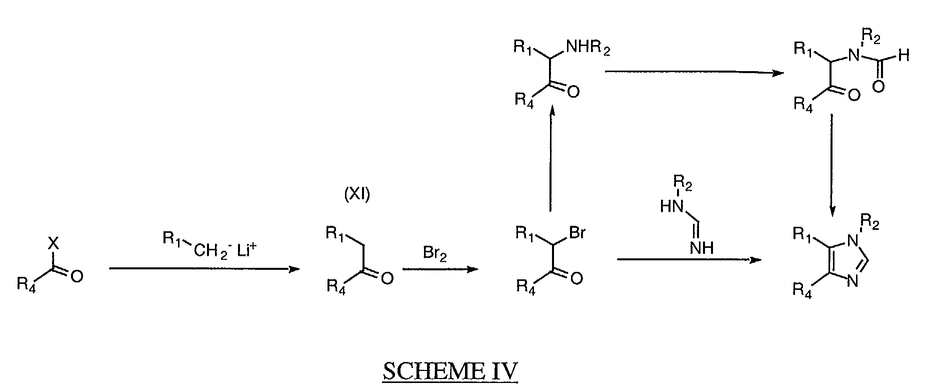

In a further process, **N**-substituted compounds of Formula (I) may be prepared by treating the anion of an amide of Formula (XII):

R₁CH₂NR₂COH (XII)

wherein R₁ and R₂ with:
(a) a nitrile of the Formula (XIII):

   R₄CN (XIII)

   wherein R₄ is as hereinbefore defined, or
(b) an excess of an acyl halide, for instance an acyl chloride, of the Formula (XIV):

   R₄COHal (XIV)

   wherein R₄ is as hereinbefore defined and Hal is halogen, or a corresponding anhydride, to give a *bis*-acylated intermediate which is then treated with a source of ammonia, such as ammonium acetate.

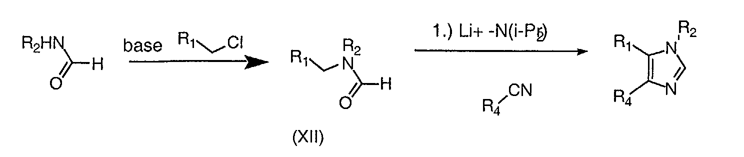

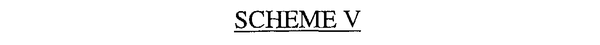

One variation of this approach is illustrated in Scheme V above. A primary amine (R₂NH₂) is treated with a halomethyl heterocycle of Formula R₁CH₂X to give the secondary amine which is then converted to the amide by standard techniques. Alternatively the amide may be prepared as illustrated in scheme V by alkylation of the formamide with R₁CH₂X. Deprotonation of this amide with a strong amide base, such as lithium di-*iso*-propyl amide or sodium *bis*-(trimethylsilyl)amide, followed by addition of an excess of an aroyl chloride yields the *bis*-acylated compound which is then closed to an imidazole compound of Formula (I), by heating in acetic acid containing ammonium acetate. Alternatively, the anion of the amide may be reacted with a substituted aryl nitrile to produce the imidazole of Formula (I) directly.

Suitable protecting groups for use with hydroxyl groups and the imidazole nitrogen are well known in the art and described in many references, for instance, Protecting Groups in Organic Synthesis, Greene T W, Wiley-Interscience, New York, 1981. Suitable examples of hydroxyl protecting groups include silyl ethers, such as t-butyldimethyl or t-butyldiphenyl, and alkyl ethers, such as methyl connected by an alkyl chain of variable link, (CR₁₀R₂₀)ₙ. Suitable examples of imidazole nitrogen protecting groups include tetrahydropyranyl.

Pharmaceutically acid addition salts of compounds of Formula (I) may be obtained in known manner, for example by treatment thereof with an appropriate amount of acid in the presence of a suitable solvent.

In the Examples, all temperatures are in degrees Centigrade (°C). Mass spectra were performed upon a VG Zab mass spectrometer using fast atom bombardment, unless otherwise indicated. ¹H-NMR (hereinafter "NMR") spectra were recorded at 250 MHz using a Bruker AM 250 or Am 400 spectrometer. Multiplicities indicated are: s=singlet, d=doublet, t=triplet, q=quartet, m=multiplet and br indicates a broad signal. Sat. indicates a saturated solution, eq indicates the proportion of a molar equivalent of reagent relative to the principal reactant.

Flash chromatography is run over Merck Silica gel 60 (230 - 400 mesh).

### SYNTHETIC EXAMPLES

The invention will now be described by reference to the following examples which are merely illustrative and are not to be construed as a limitation of the scope of the present invention. All temperatures are given in degrees centigrade, all solvents are highest available purity and all reactions run under anydrous conditions in an argon atmosphere unless otherwise indicated.

### Example 1

### 1-[3-(4-Morpholinyl)propyl]-4-(4-fluorophenyl)-5-(4-pyridyl)imidazole

### a) 4-fluorophenyl-tolylthiomethylformamide

A solution of p-fluorobenzaldehyde (13.1 milliliters (hereinafter mL), 122 millimoles (hereinafter mmol) thiocresol (16.64 grams (hereinafter g), 122 mmol), formamide (15.0 mL, 445 mmol), and toluene (300 mL) were combined and heated to toluene reflux with azeotropic removal of H₂O for 18 h. The cooled reaction was diluted with EtOAc (500 mL) and washed with satd aq Na₂CO₃(3 x 100 mL), satd aq NaCl (100 mL), dried (Na₂SO₄), and concentrated. The residue was triturated with petroleum ether, filtered and dried in vacuo to afford 28.50 g of the title compound as a white solid (85 %). melting point (hereinafter mp) = 119 - 120°.

### b) 4-fluorophenyl-tolylthiomethylisocyanide

The compound of example 1(a) (25 g, 91 mmol) in CH₂Cl₂ (300 mL) was cooled to -30° and with mechanical stirring POCl₃ (11 mL, 110 mmol) was added dropwise followed by the dropwise addition of Et₃N (45 mL, 320 mmol) with the temperature maintained below -30°. Stirred at -30° for 30 min and 5° for 2 h, diluted with CH₂Cl₂ (300 mL) and washed with 5% aq Na₂CO₃ (3 x 100 mL), dried (Na₂SO₄) and concentrated to 500 mL. This solution was filtered through a 12 x 16 cm cylinder of silica in a large sintered glass funnel with CH₂Cl₂ to afford 12.5 g (53%) of purified isonitrile as a light brown, waxy solid. IR (CH₂Cl₂) 2130 cm⁻¹.

### c) Pyridine-4-carboxaldehyde [4-Morpholinylprop-3-yl]imine

Pyridine-4-carboxaldehyde (2.14 g, 20 mmoL), 4-(3-aminopropyl)morpholine (2.88 g, 20 mmol), toluene (50 mL) and MgSO₄ (2 g) were combined and stirred under argon for 18 h. The MgSO₄ was filtered off and the filtrate was concentrated and the residue was reconcentrated from CH₂Cl₂ to afford 4.52 g (97%) of the title compound as a yellow oil containing less than 5% of aldehyde based on ¹H NMR. ¹H NMR (CD₃Cl): d 8.69 (d, J = 4.5 Hz, 2H), 8.28 (s, 1H), 7.58 (d, J = 4.5 Hz, 2H), 3.84 (m, 6H), 2.44 (m, 6H), 1.91 (m, 2H).

### d) 1-[3-(4-Morpholinyl)propyl]-4-(4-fluorophenyl)-5-(4-pyridyl)imidazole

The compound of example 1(b) (1.41 g, 5.5 mmol), and the compound of example 1(c) (1.17 g, 5.0 mmol) and CH₂Cl₂ (10 mL) were cooled to 5 °C. 1.5.7-triazabicyclo[4.4.0]dec-5-ene, henceforth referred to as TBD, (0.71 g 5.0 mmol) was added and the reaction was kept at 5 °C for 16 h, diluted with EtOAc (80 mL) and washed with satd aq Na₂CO₃ (2 x 15 mL). The EtOAc was extracted with 1 N HCl (3 x 15 mL), and the acid phases were washed with EtOAc (2 x 25 mL), layered with EtOAc (25 mL) and made basic by the addition of solid K₂CO₃ til pH 8.0 and then 10% NaOH til pH 10. The phases were separated and the aq was extracted with additional EtOAc (3 x 25 mL). The extracts were dried (K₂CO₃) concentrated and the residue was crystalized from acetone/hexane to afford 0.94 g (51%) of the title compound. mp = 149 - 150°.

### Example 2

### 1-(3-Chloropropyl)-4-(4-fluorophenyl)-5-(4-pyridyl)imidazole

### a) Pyridine-4-carboxaldehyde (3-Chloropropyl)imine

To 3-chloropropylamine HCl (15.1 g, 0.120 moles (hereinafter mol)) and H₂O (100 mL) was added pyridine-4-carboxaldehyde (9.55 mL, 0.100 mol), then K₂CO₃ (8.28 g, 0.060 mol) then CH₂CL₂ (100 mL) and the mixture was stirred for 40 min. The phases were separated and the aq phase was extracted with additional CH₂Cl₂ (2 x 50 mL), dried (Na₂SO₄) and concentrated to afford 17.1 g (94%) ¹H NMR (CD₃Cl): d 8.69 (d, J = 4.5 Hz, 2H, 8.32 (s, 1H), 8.28 (s, 1H), 7.58 (d, J = 4.5 Hz, 2H), 3.71 (m, 2H), 3.63 (t, J = 6Hz, 2H), 2.24 (t, J = 6Hz, 2H). The presence of 9% of the aldehyde was evident by ¹H NMR.

### b) 1-(3-Chloropropyl)-4-(4-fluorophenyl)-5-(4-pyridyl)imidazole

The compound of example 1(b) (6.85 g, 26.6 mmol), the compound of example 2(a) (6.32g, 34.6 mmol), CH₂Cl₂ (70 mL), and TBD (4.07 g, 28.4 mmol) were reacted by the procedure of example 1(d) to afford 3.19 g (38%). mp = 139 - 140°.

### Example 3

### 1-(3-Azidopropyl)-4-(4-fluorophenyl)-5-(4-pyridyl)imidazole

To a solution of the compound of example 2(b) (250 milligram (hereinafter mg), 0.79 mmol) and DMF (5 mL) was added NaN₃ (256 mg, 3.95 mmol) and NaI (12 mg, 0.08 mmol) and the mixture was heated to 90° till the reaction was completed based on tlc analysis (19:1 CH₂Cl₂/MeOH). The cooled reaction was added to 5% aq Na₂CO₃ (20 mL) and extracted with EtOAc (3 x 25 mL). The combined extracts were washed with H₂O (3 x 25 mL) and flash chromatographed (2.2 x 10 cm column) with 0 - 1% MeOH in CH₂Cl₂ to afford 254 mg (100%) of the title compound as a white solid. mp = 64 - 65°.

### Example 4

### 1-(3-Aminopropyl)-4-(4-fluorophenyl)-5-(4-pyridyl)imidazole

The compound of example 3, described above (254 mg, 0.79 mmol), was dissolved in THF (2 mL) an added dropwise to a 0° solution of 1 N LiAlH₄ in THF (1.2 mL, 1.2 mmol), stirred at 0° for 15 min, EtOAc (4 mL) was carefully added and the mixture was added to ice cold 10% NaOH (15 mL) and the product was extracted with EtOAc (4 x 25 mL), dried (K₂CO₃) and concentrated to a waxy solid, (175 mg, 75%). mp = 81 - 82°.

### Example 5

### 1-(3-Methylsulfonamidopropyl)-4-(4-fluorophenyl)-5-(4-pyridyl)imidazole

To the compound of Example 4, described above (79 mg, 0.26 mmol) in CH₂Cl₂ (0.5 mL) was added Et₃N (72 uL, 0.52 mmol), and then methanesulfonyl chloride (25 uL, 0.31 mmol). The reaction exothermed to CH₂Cl₂ reflux briefly. The reaction was over within 1 min based on tlc (19 : 1 CH₂Cl₂ /MeOH) and was poured into 10% NaOH (5 mL) and extracted with EtOAc (3 x 20 mL). The extracts were washed with H₂O (10 mL) and satd aq Nacl (10 mL), dried (Na₂SO₄), concentrated and flash chromatographed (1 x 10 cm silica) with 0 - 8% MeOH in CH₂Cl₂ to afford 63 mg (65%). mp = 186 - 187°.

### Example 6

### 1-[3-(N-Phenylmethyl)aminopropyl]-4-(4-fluorophenyl)-5-(4-pyridyl)imidazole

Following the procedure of example 3 above, except using benzylamine as the nucleophile and purification of the crude product by trituration with hot hexane, the title compound was prepared as a white solid (32% yield). mp = 125 - 126°.

### Example 7

### 1-[3-(N-Phenylmethyl-N-methyl)aminopropyl)-4-(4-fluorophenyl)-5-(4-pyridyl)imidazole

Following the procedure of example 3 except using N-benzylmethylamine as the nucleophile and purification of the crude product by trituration with hot hexane, the title compound was prepared as a white solid (42% yield). mp = 90 - 91°.

### Example 8

### 1-[3-(1-Pyrrolidinyl)propyl]-4-(4-fluorophenyl)-5-(4-pyridyl)imidazole

Following the procedure of example 3 except using pyrrolidine as the nucleophile and purification of the crude product by trituration with hot hexane, the title compound was prepared as a white solid (35% yield). mp = 105 - 107°.

### Example 9

### 1-(3-Diethylaminopropyl)-4-(4-fluorophenyl)-5-(4-pyridyl)imidazole

Following the procedure of example 3 except using diethylamine as the nucleophile and isolation of the product by extraction with diethyl ether, the title compound was prepared as a white solid (21% yield). mp = 94 - 95°.

### Example 10

### 1-[3-(1-Piperidinyl)propyl]-4-(4-fluorophenyl)-5-(4-pyridyl)imidazole

Following the procedure of example 3 except using piperidine as the nucleophile and purification of the crude product by trituration with hot hexane, the title compound was prepared as a white solid (63% yield). mp = 105 - 108°.

### Example 11

### 1-[3-(Methylthio)propyl]-4-(4-fluorophenyl)-5-(4-pyridyl)imidazole

Following the procedure of example 3 except using sodium thiomethane as the nucleophile and omitting the sodium iodide followed by purification of the crude product by trituration with hot hexane, the title compound was prepared as a white solid (50% yield). mp = 85 - 86°.

### Example 12

### 1-[2-(4-Morpholinyl)ethyl]-4-(4-fluorophenyl)-5-(4-pyridyl)imidazole

### a) Pyridine-4-carboxaldehyde-[2-(4-Morpholinyl)ethyl]imine

Following the procedure of example 1(c) except using 4-(2-aminoethyl)morpholine as the amine, the title compound was prepared as a light yellow oil (100%) containing less than 10% of aldehyde based on ¹H NMR. ¹H NMR (CD₃Cl): d 8.68 (d, J = 6 Hz, 2H), 8.28 (s, 1H), 7.58 (d, J = 6 Hz, 2H), 3.82 (m, 2H), 3.72 (m, 4H), 2.72 (m. 2H), 2.55 (m, 4H).

### b) 1-[2-(4-Morpholinyl)ethyl]-4-(4-fluorophenyl)-5-(4-pyridyl)imidazole

Following the procedure of example 1(d) except using the compound of example 20(a) as the imine, afforded the title compound as a white solid (21%). mp = 114-115°.

### Example 13

### 1-[3-(4-Morpholinyl)propyl]-4-(3-methylthiophenyl)-5-(4-pyridyl)imidazole

### a) N-[3-methylthiophenyl-(tolylthio)methyl]formamide

Following the procedure of example 1(a) except using m-methylthiobenzaldehyde as the aldehyde, the title compound was prepared as a white solid (73%). mp = 103 - 104°.

### b) 3-methylthiophenyl-(tolylthio)methylisocyanide

Following the procedure of example 1(b) except using the compound of the previous step as the formamide, the title compound was prepared as a light brown oil (77%). IR (CH₂Cl₂) 2120 cm⁻¹.

### c) 1-[3-(4-Morpholinyl)propyl]-4-(3-methylthiophenyl)-5-(4-pyridyl)imidazole

Following the procedure of example 1(d) except using the compound of the previous step as the isonitrile, afforded the title compound as a white solid (31%). mp = 105 - 106°.

### Example 14

### 1-[3-(4-Morpholinyl)propyl]-4-(3-methylsulfinylphenyl)-5-(4-pyridyl)imidazole

The compound of example 13(c) (200 mg, 0.49 mmol) was dissolved in HOAc (4 mL). K₂S₂O₈ (151 mg, 0.56 mmol) dissolved in H₂O was added and the solution was stirred for 16 h, poured into 10 % aq NaOH (50 mL) (the resulting solution was > pH 10) and extracted with EtOAc (3 x 25 mL). The extracts were dried (K₂CO₃), concentrated and the residual oil crystalized from acetone/hexane to afford 87 mg (42%) of a white solid. mp = 117 - 118°.

### Example 15

### 1-[3-(N-methyl-N-benzyl)aminopropyl]-4-(3-methylthiophenyl)-5-(4-pyridyl)imidazole

### a) Pyridine-4-carboxaldehyde 3-(N-methyl-N-benzylaminopropyl)imine

Following the procedure of example 1(c) except using 3(-N-Methyl-N-benzylamino)propylamine as the amine (Ueda,T.; Ishizaki,K.; Chem. Pharm. Bull. 1967, 15, 228 - 237.), the title compound was obtained as a light yellow oil (100%) containing less than 10% of aldehyde based on ¹H NMR. ¹H NMR (CD₃Cl): d 8.65 (d, J = 7 Hz, 2H), 8.21 (s, 1H), 7.54 (d, J = 4.5 Hz, 2H), 7.52 (m, 5H), 3.69 (t, J = 11 Hz, 2H), 3.48(s, 2H), 2.44 (t, J = 11 Hz, 2H), 2.18 (s, 3H), 1.91 (m, 2H).

### b) 1-[3-(N-methyl-N-benzyl)aminopropyl]-4-(3-methylthiophenyl)-5-(4-pyridyl)imidazole

Following the procedure of example 1(d) except using the compound of example 13(b) as the isonitrile, and the compound prepared in the previous step as the imine afforded the title compound as a white solid (36%). mp = 87 - 88°.

### Example 16

### 1-[3-(N-methyl-N-benzyl)aminopropyl]-4-(3-methylsulfinylphenyl)-5-(4-pyridyl)imidazole

Following the procedure of example 14 except using the compound of example 15(b) as the sulfide afforded the title compound as a white solid (97%). mp = 84 - 85°.

### Example 17

### 1-[4-(Methylthio)phenyl]-4-(4-fluorophenyl)-5-(4-pyridyl)imidazole

### a) Pyridine-4-carboxaldehdye (4-methylthiophenyl)imine

Following the procedure of example 1(c) except using 4-(methylthio)aniline as the amine afforded (100%) of a light yellow oil with no detectable amount of aldehyde based on ¹H NMR. ¹H NMR (CD₃Cl): d 8.75 (d, J = 6 Hz, 2H), 8.47 (s, 1H), 7.74 (d, J = 6 Hz, 2H), 7.30 (d, J = 8 Hz, 2H), 7.22 (d, J = 8 Hz, 2H), 2.52(s, 3H).

### b) 1-[4-(Methylthio)phenyl]-4-(4-fluorophenyl)-5-(4-pyridyl)imidazole

Following the procedure of example 1(d) except using the compound of the previous step as the imine afforded the title compound as a white solid (27%). mp = 172-173°.

### Example 18

### 1-[4-(Methylsulfinyl)phenyl]-4-(4-fluorophenyl)-5-(4-pyridyl)imidazole

Following the procedure of example 14 except using the compound of example 17(b) as the sulfide afforded the title compound as a white solid (67%). mp = 202 - 203°.

### Example 19

### 1-[3-(Methylthio)phenyl]-4-(4-fluorophenyl)-5-(4-pyridyl)imidazole

### a) Pyridine-4-carboxaldehdye (3-methylthiophenyl)imine

Following the procedure of example 1(c) except using 3-(methylthio)aniline as the amine afforded (98%) of a light yellow oil with *ca* 2.5% of aldehyde based on ¹H NMR. ¹H NMR (CD₃Cl): d 8.76 (d, J = 6 Hz, 2H), 8.44 (s, 1H), 7.74 (d, J = 6 Hz, 2H), 7.30 (d, J = 8 Hz, 2H), 7.34 - 6.98 (m, 4H), 2.52(s, 3H).

### b) 1-[3-(methylthio)phenyl]-4-(4-fluorophenyl)-5-(4-pyridyl)imidazole

Following the procedure of example 1(d) except using the compound of the previous step as the imine afforded the title compound as a white solid (42%). mp = 155-156°.

### Example 20

### 1-[3-(Methylsulfinyl)phenyl]-4-(4-fluorophenyl)-5-(4-pyridyl)imidazole

Following the procedure of example 14 except using the compound of example 19(b) as the sulfide afforded the title compound as a white solid (67%). mp = 233 - 234°.

### Example 21

### 1-[2-(Methylthio)phenyl]-4-(4-fluorophenyl)-5-(4-pyridyl)imidazole

### a) Pyridine-4-carboxaldehdye (2-methylthiophenyl)imine

Following the procedure of example 1(c) except using 2-(methylthio)aniline as the amine afforded (98%) of a light yellow oil with *ca* 8% of aldehyde based on ¹H NMR. ¹H NMR (CD₃Cl): d 8.75 (d, J = 6 Hz, 2H), 8.41 (s, 1H), 7.79 (d, J = 6 Hz, 2H), 7.36 - 7.00 (m, 4H), 2.47(s, 3H).

### b) 1-[2-(methylthio)phenyl]-4-(4-fluorophenyl)-5-(4-pyridyl)imidazole

Following the procedure of example 1(d) except using the compound of the previous step as the imine and purification by flash chromatography with 0 - 1% MeOH in CH₂Cl₂ afforded the title compound as a non-crystalline white foam (53%). mp = 59 - 60°.

### Example 22

### 1-[2-(Methylsulfinyl)phenyl]-4-(4-fluorophenyl)-5-(4-pyridyl)imidazole

Following the procedure of example 14 except using the compound of example 21(b) as the sulfide, and purification by flash chromatography with 0 - 4% MeOH in CH₂Cl₂ afforded the title compound as a non-crystalline white foam (52%). mp = 60 - 165°. (The ill defined mp is probably the result of a mixture of conformational isomers which is clearly indicated in the ¹H and ¹³C NMR spectra of this compound.)

### Example 23

### 1-(3-Chloropropyl)-4-(4-fluorophenyl)-5-(4-pyridyl)imidazole

### (See also Example 2 above for alternative method of preparation)

### a) 4-Fluorophenyl-tosylmethylformamide

To a solution of toluene sulphinic acid sodium salt hydrate (120g) in water (750 ml) was added concentrated sulphuric acid (16ml). Dichloromethane (500ml) was added and the organic and aqueous layers were separated; the aqueous layers being extracted with dichloromethane (2x200ml). The combined organic extracts were dried (Na₂S0₄) and evaporated to dryness to yield the solid sulphinic acid (71.79g, 0.46 mole). This was added to p-fluorobenzaldehyde (57.04g, 0.46 mole) and formamide (62.1g, 1.38 mole) and the resulting mixture was stirred with camphor-10-sulphonic acid (21.3g, 0.092 mole) at 60-65°, under nitrogen for 22 hours. A solution of sodium bicarbonate (33.6g, 0.40 mole) in water (400ml) was added to the ice-cooled solid product which was broken up and stirred for 30 minutes. The crude product was collected and washed with acetone (220ml) and then ether (3x220ml) and dried to yield the desired product, 91.5g, 64.8%.

### b) 4-Fluorophenyl-tosylmethylisocyanide

To a suspension of the compound of the previous step (3.22g, 10.5 mmole) in dimethoxyethane (21ml) stirring at -10° was added phosphorus oxychloride (2.36ml, 25.3 mmole) dropwise over 5 minutes. Triethylamine (7.35ml, 52.5 mmole) was then added dropwise over 10 minutes and the reaction mixture was poured into saturated sodium bicarbonate solution (100ml) and the oily product was extracted into dichloromethane (2x30ml). The organic extracts were evaporated to yield a black oil (3.51g) which was eluted from Grade III basic alumina (60g) using dichloromethane. The combined product fractions were evaporated and ether added to crystallize the desired product, 1.735g, 57%.

### c) 1-(3-Chloropropyl)-4-(4-fluorophenyl)-5-(4-pyridyl)-imidazole

To a solution of the compound of the previous step (1.183g, 4.09mmol) and the compound of example 2(a) (1.122g, 6.15mmole) in dimethoxyethane (15ml) at ambient temperature was added dropwise over 10 minutes a solution of DBU (0.67ml, 4.51mmole) in dimethoxyethane (10ml). The reaction mixture was stirred at ambient temperature for 1-1/2hours and then evaporated to leave an oil which was eluted from Grade III basic alumina (100g) to yield the desired product, 1.096g, 85%.

### Example 24

### 1-[4-(4-Morpholinyl)butyl]-4-(4-fluorophenyl)-5-(4-pyridyl)imidazole

### a) 4-(4-Morpholino)butyl-1-pthalimide

4-Bromobutyl-1-pthalimide (5.0 g, 17.7 mmol) and morpholine (20 mL) were combined and stirred for 3 h, diluted with Et₂O (200 mL), and filtered. The solid was washed with more Et₂O and the combined filtrates were extracted with 3 N HCl (3 x 25 mL). The combined acid phases were washed with Et₂O (3 x 50 mL), layered with EtOAc and made basic by the addition of solid K₂CO₃ til the foaming stopped and then 10% aq NaOH was added til the pH was > 10. Extraction with EtOAc (3 x 100 mL), drying, (K₂CO₃), concentration and flash filtration 1 L silica wash first with 0 - 4% MeOH in CH₂Cl₂ and then elute product with 4% MeOH and 1% Et₃N in CH₂Cl₂ to afford 5.52 g (54%) of the title compound as a white solid.

### b) 4-(4-Morpholino)butylamine

The compound of example 24 (a) (1.0 g, 3.47 mmol), hydrazine monohydrate (190 µl, 3.82 mmol) and CH₃OH (20 mL) were combined and stirred at 23° overnight. The CH₃OH was removed in vacuo and the resudue was concentrated to dryness from EtOH. The residue was combined with 2N HCl (20 mL) and stirred for 2h, filtered and the solid was washed with H₂O. The combined filtrates were concentrated in vacuo and reconcentrated from EtOH twice to give a white foam which was dissolved in 3:1 CH₂Cl₂/CH₃OH, and stirred with solid K₂CO₃ for 5 min and filtered. The filtrate was concentrated to afford 0.535 g (80%) of a brown oil. ¹H NMR (CD₃Cl): 3.7 - 3.2 (m, 6H), 2.7 - 2.2 (m, 6), 1.6 - 1.3 (m, 6H).

### c) Pyridine-4-carboxaldehyde [4-(4-morpholinyl)butyl]imine

Following the procedure of example 1(c) except using the compound of example 24(b) as the amine the title compound was prepared as a light yellow oil (100%) containing 30% of aldehyde based on ¹H NMR. ¹H NMR (CD₃Cl): 8.60 (d, J = 6 Hz, 2H), 8.19 (s, 1H), 7.51 (d, J = 6 Hz, 2H), 3.7 - 3.2 (m, 6H), 2.5 - 2.2 (m, 6), 1.7 - 1.4 (m, 4H).

### d) 1-[4-(4-Morpholinyl)butyl]-4-(4-fluorophenyl)-5-(4-pyridyl)imidazole

Following the procedure of example 1(d) except using the compound of example 24(c) as the imine afforded the title compound (38%). mp = 103 - 104°.

### Example 25

### 1-Cyclopropyl-4-(4-fluorophenyl)-5-(4-pyridyl)imidazole

### a) Pyridine-4-carboxaldehyde cyclopropylimine

Following the procedure of example 1(c) except using a 100% excess of the volatile cyclopropylamine the title compound was prepared as a light yellow oil (100%). ¹H NMR (CD₃Cl): 8.65 (d, J = 6 Hz, 2H), 8.40 (s, 1H), 7.51 (d, J = 6 Hz, 2H), 3.07 (m, 1H), 1.01 (m, 4H)

### b) 1-Cyclopropyl-4-(4-fluorophenyl)-5-(4-pyridyl)imidazole

The compound of the previous step (20 mmol), the compound of example 1(b) (5.65g, 22 mmol), and CH₂Cl₂ (20 mL) were cooled to 0° and TBD (2.84g, 20 mmol) was added. Stirred at 5° for 2h, 23° for 48h and refluxed for 4 h. The crude reaction was flash filtered through a sintered glass funnel filled with silica (1 L of silica) eluting with 0 - 4% CH₃OH in CH₂Cl₂. Crystals from hexane/acetone to afford 839 mg (15%) mp = 129.0 - 129.5°.

### Example 26

### 1-Isopropyl-4-(4-fluorophenyl)-5-(4-pyridyl)imidazolea) Pyridine-4-carboxaldehyde isopropylimine

Following the procedure of example 1(c) except using isopropylamine as the amine the title compound was prepared as a light yellow oil (100%). ¹H NMR (CD₃Cl): 8.67 (d, J = 4.4 Hz, 2H), 8.27 (s, 1H), 7.59 (d, J = 4.43 Hz, 2H), 3.59 (m, 1H), 1.27 (d, J = 6.3 Hz, 6H).

### b) 1-Isopropyl-4-(4-fluorophenyl)-5-(4-pyridyl)imidazole

Following the procedure of example 1(d) and substituting the imine of the previous step the compound was prepared using a modified work up of flash filtration of the crude reaction through silica (0 - 4% CH₃OH in CH₂Cl₂). Two crystallizations from hexane /acetone afforded the title compound as tan needles (30 %). mp = 179.0 - 179.5.

### Example 27

### 1-Cyclopropylmethyl-4-(4-fluorophenyl)-5-(4-pyridyl)imidazole

### a) Pyridine-4-carboxaldehyde Cyclopropylmethylimine

Following the procedure of example 1(c) except using cyclopropylmethylamine as the amine the title compound was prepared as a light yellow oil (100%). ¹H NMR (CD₃Cl): 8.69 (d, J = 4.5 Hz, 2H), 8.27 (s, 1H), 7.61 (d, J = 4.5 Hz, 2H), 3.55 (d, J = 6.7 Hz, 2H), 1.15 (m,1H), 0.57 (m, 2H), 0.27 (m, 2H).

### b) 1-Cyclopropylmethyl-4-(4-fluorophenyl)-5-(4-pyridyl)imidazole

Following the procedure of example 1(d) substituting the imine of the previous step the compound was prepared using a modified work up of flash filtration of the crude reaction through silica (0 - 4% CH₃OH in CH₂Cl₂). Crystallization from hexane /acetone afforded the title compound as white flakes (62 %). mp = 162.0 - 162.5.

### Example 28

### 1-tert-Butyl-4-(4-fluorophenyl)-5-(4-pyridyl)imidazole

### a) Pyridine-4-carboxaldehyde tert-butylimine

Following the procedure of example 1(c) except using tert-butylamine as the amine the title compound was prepared as a light yellow oil (100%). ¹H NMR (CD₃Cl): 8.67 (d, J = 4.4 Hz, 2H), 8.22 (s, 1H), 7.61 (d, J = 4.4 Hz, 2H), 1.30 (s, 9H).

### b) 1-tert-Butyl-4-(4-fluorophenyl)-5-(4-pyridyl)imidazole

Following the procedure of example 1(d) substituting the imine of the previous step the compound was prepared using a modified work up of flash filtration of the crude reaction through silica (0 - 4% CH₃OH in CH₂Cl₂) to afford the title compound as tan powder (16 %). mp = 199.0 - 200.0.

### Example 29

### 1-(2,2-Diethoxyethyl)-4-(4-fluorophenyl)-5-(4-pyridyl)imidazole

### a) Pyridine-4-carboxaldehyde 2,2-diethoxyethylimine

Following the procedure of example 1(c) except using 2,2-diethoxyethylamine as the amine the title compound was prepared as a light yellow oil (100%). ¹H NMR (CD₃Cl): 8.69 (d, J = 4.4 Hz, 2H), 8.28 (s, 1H), 7.60 (d, J = 4.4 Hz, 2H), 4.82 (t, J = 5.1 Hz, 1H0, 3.82 (d, J = 5.1 Hz, 1H), 3.72 (m, 2H), 3.57 (m, 2H), 1.21 (t, J = 7.3 Hz, 6H).

### b) 1-(2,2-Diethoxyethyl)-4-(4-fluorophenyl)-5-(4-pyridyl)imidazole

Following the procedure of example 1(d) substituting the imine of the previous step, the compound was prepared using a modified work up of flash filtration of the crude reaction through silica (0 - 4% CH₃OH in CH₂Cl₂), followed by a flash chromatography through silica with 25 - 100% EtOAc in hexane) and trituration of the resulting gum with hexane afforded the title compound as a white powder (47 %). mp = 69.5 - 70.0.

### Example 30

### 1-Formylmethyl-4-(4-fluorophenyl)-5-(4-pyridyl)imidazole

The product of example 29(b) (400 mg, 1.13 mmol), H₂O (10 mL), acetone (10 mL) and concd H₂SO₄ (1 mL) were combined and refluxed for 24 h. Most of the acetone was removed in vacuo and the residue was combined with 5% aq Na₂CO₃ and extracted with EtOAc, dried (Na₂SO₄), concentrated and crystallyzed from acetone to afford the title compound as a white powder (47 %). mp = 118.5 - 119.0.

### Example 31

### 1-Hydroxyiminylmethyl-4-(4-fluorophenyl)-5-(4-pyridyl)imidazole

The product of example 30 (317 mg, 1.13 mmol), hydroxylamine hydrochloride (317 mg), pyridine (317 µL), and EtOH (3.8 mL) were combined and refluxed for 3h, poured into 5% aq Na₂CO₃, and extracted with EtOAc, dried (Na₂SO₄) and flash filtered in 0 - 4% MeOH in CH₂Cl₂ to afford 261 mg (78%) of the title compound as a white powder. mp = 184.0 - 185.0.

### Example 32

### 1-Cyanomethyl-4-(4-fluorophenyl)-5-(4-pyridyl)imidazole

The product of example 31 (250 mg, 0.84 mmol), and CuSO₄ were combined and refluxed for 2h. The cooled reaction was flash filtered in 0 - 4% MeOH in CH₂Cl₂ to afford 129 mg (55%) of the title compound as a white powder. mp = 132.0 - 133.0.

### Example 33

### 1-[3-(4-Morpholinyl)propyl)-4-(4-fluorophenyl)-5-(2-methylpyrid-4-yl)imidazolea) 4-Formyl-2-methylpyridine

4-Cyano-2-methylpyridine was prepared from 2,6-lutidine asccording to the literature procedure (Yamanaka,H.; Abe,H.; Sakamoto,T.; Hidetoshi, Hiranuma,H.; Kamata,A. Chem. Pharm. Bull. 25(7), 1821 - 1826.). A solution of 4-Cyano-2-methylpyridine (0.367 g, 3.11 mmoL) and toluene (3.5 mL) was cooled to -78° and 1 M DIBAL in hexanes (3.6 mL, 3.6 mmoL) was added dropwise via syringe pump (T < -65°). The reaction was warmed to 5° and stirred for 5 min, recooled to -78° and CH₃OH (3.5 mL) was added (T < -40°), warmed to 5° and stirred for 5 min and then 25% aq Rochelle's salt was added, stirred for 3 min and then acidified to < pH 1.0 with 10% aq H₂SO₄. The aqueous was made basic by the addition of solid K₂CO₃ and extracted with EtOAc. The extracts were dried (Na₂SO₄) concentrated and filtered through silica (2% MeOH in CH₂Cl₂) to afford 253 mg (84%) of aldehyde. H¹ NMR (CD₃Cl): d 10.05 (s, 1H), 8.74 (d, J = 7Hz, 1 H), 7.51 (s, 1H), 7.30 (d, J = 7Hz, 1H), 2.68 (s, 3H).

### b) Pyridine-4-carboxaldehyde [3-(4-morpholinyl)propyllimine

The product of the previous step and 4-(3-aminopropyl)morpholine were reacted by the procedure of example 1(c) to afford the title compound as a yellow oil containing no aldehyde based on ¹H NMR. ¹H NMR (CD₃Cl): d 8.57 (d, J = 5.0 Hz, 1H), 8.25 (s, 1H), 7.46 (s, 1H); 7.36 (d, J = 5.0 Hz, 1H), 3.71 (m, 6H), 2.60 (s, 1H); 2.35 (m, 6H), 1.90 (m, 2H).

### c) 1-[3-(4-Morpholinyl)propyl)-4-(4-fluorophenyl)-5-(2-methylpyrid-4-yl)imidazole

The compound of the previous step, and the compound of example 1(b) were reacted by the procedure of example 1(d) to afford the title compound as a white solid [51% from 33 (a)]. mp = 116 - 117°

### Example 34

### 4-(4-Fluorophenyl)-1-[3-(4-morpholinyl)propyl]-5-(2-chloropyridin-4-yl) imidazole a) 2-Chloropyridine-4-carboxaldehyde [3-(4-morpholinyl)propyl]imine

2-Chloropyridine-4-carboxaldehyde was prepared as described in the patent literature (WPI Acc. No. 88-258820/37) whose disclosure is incorporated by reference in its entirety herein. This aldehyde was reacted with 4-(3-aminopropyl)morpholine by the procedure of example 1(c) to afford the title compound as a yellow oil.¹H NMR (CD₃Cl): δ 8.45 (d, J = 5.1 Hz, 1H), 8.24 (s, 1H), 7.63 (s, 1H); 7.51 (d, J = 5.1 Hz, 1H), 3.72 (m, 6H), 2.44 (m, 6H), 1.91 (m, 2H).

### b) 4-(4-Fluorophenyl)-1-[3-(4-morpholinyl)propyl]-5-(2-chloro-4-pyridinyl)imidazole

Following the procedure of example 1(d) except using the compound of the previous step as the imine afforded the title compound as a white solid (93%). mp = 97.0-97.5°.

### Example 35

### 4-(4-Fluorophenyl)-1-[3-(4-morpholinyl)propyl]-5-(2-amino-4-pyridinyl)imidazole

### a) 4-(4-Fluorophenyl)-1-[3-(4-morpholinyl)propyl]-5-(2-hydrazinyl-4-pyridinyl) imidazole

The compound of example 34(b) (872 mg, 2.18 mmoL) and 98% hydrazine hydrate (9 mL) was heated to 115° (bath temp) for 20 h, cooled to 23° combined with H₂O (20 mL) and extracted with EtOAc (3 x 25 mL). The combined extracts were washed with H₂O (2 x 20 mL) and dried (Na₂SO₄). Flash chromatography with 0 - 8% CH₃OH in CH₂Cl₂ afforded 547 mg (63%) the title compound as a white solid.

### b) 4-(4-Fluorophenyl)-1-[3-(4-morpholinyl)propyl]-5-(2-amino-4-pyridinyl) imidazole

The compound of the previous step (100 mg, 0.25 mmoL), absolute EtOH (15 mL) and Raney Ni (0.4 mL) were shaken under H₂ (45 psi) for 4 h. Flash chromatography with 0-8% CH₃OH in CH₂Cl₂ afforded 34 mg (37%) the title compound as a white solid. mp = 186 - 187°.

### Example 36

### 1-(4-Carboxymethyl)propyl-4-(4-fluorophenyl)-5-(4-pyridyl)imidazole

### a) Pyridine-4-carboxaldehyde (4-carboxymethylbutyl)imine

Pyridine-4-carboxaldehyde was reacted with methyl-4-aminobutyrate by the procedure of example 1(c) to afford the title compound as a yellow oil. ¹H NMR (CD₃Cl): δ 8.69 (d, J = 5.8 Hz, 2H), 8.27 (s, 1H); 7.56 (d, J = 5.8 Hz, 2H), 3.70 (m, 2H); 2.31 (t, J = 8.0 Hz, 2H), 2.08 (m, 2H).

### b) 1-(4-Carboxymethyl)propyl-4-(4-fluorophenyl)-5-(4-pyridyl)imidazole

Following the procedure of example 1(d) except using the compound of the previous step as the imine afforded the title compound as a white solid (35%). mp = 69.0 - 70.0°.

### Example 37

### 1-(4-Carboxypropyl)-4-(4-fluorophenyl)-5-(4-pyridyl)imidazole

The compound of example 36 (100 mg, 0.29 mmol), CH₃OH (3 mL), and THF (1.5 mL) were combined and the resulting soln was treated with a soln of LiOH (62 mg, 1.5 mmol) in H₂O (1.5 mL) and the resulting soln was stirred for 4 h. Removal of the volatile compounents *in vacuo*, redisolving in H₂O and chromatography through HP-20 with H₂O til the eluates were neutral and then with 25% aq MeOH afforded the title compound as the lithium salt; 65mg (68%). ES (+) MS m/e = 326 (MH⁺).

### Example 38

### 1-(3-Carboxymethyl)ethyl-4-(4-fluorophenyl)-5-(4-pyridyl)imidazole

### a) Pyridine-4-carboxaldehyde (3-carboxymethyl)ethyl imine

Pyridine-4-carboxaldehyde was reacted with β-alanine methyl ester by the procedure of example 1(c) to afford the title compound as a yellow oil. ¹H NMR (CD₃Cl): δ 8.68 (d, J = 4.5 Hz, 2H), 8.33 (s, 1H); 7.57 (d, J = 4.5 Hz, 2H), 3.93 (t, J = 6.7 Hz, 2H); 3.68 (s, 3H), 2.76 (t, J = 6.7 Hz, 2H),

### b) 1-(3-Carboxymethyl)ethyl-4-(4-fluorophenyl)-5-(4-pyridyl)imidazole SB-219302

Following the procedure of example 1(d) except using the compound of the previous step as the imine afforded the title compound as a white solid (40% from the amine). mp = 119.0 - 120.0°.

### Example 39

### 1-(3-Carboxy)ethyl-4-(4-fluorophenyl)-5-(4-pyridyl)imidazole

The compound of example 38(b) was hydrolysed by the procedure of example 37 to afford the title compound as the lithium salt; (71%). ES (+) MS m/e = 312 (MH⁺).

### Example 40

### 1-(1-Benzylpiperidin-4-yl)-4-(4-fluorophenyl)-5-(4-pyridyl) imidazole

### a) Pyridine-4-carboxaldehyde (1-benzylpiperidin-4-yl)imine

Pyridine-4-carboxaldehyde was reacted with 4-amino-N-benzylpiperidine by the procedure of example 1(c) to afford the title compound as a yellow oil.

### b) 1-(1-Benzylpiperidin-4-yl)-4-(4-fluorophenyl)-5-(4-pyridyl)imidazole

Following the procedure of example 1(d) except using the compound of example the previous step as the imine afforded the title compound as a white solid (9% from the amine). ES (+) MS m/e = 413 (MH⁺).

### Example 41

### 5-(2-Aminopyrimidin-4-yl)-4-(4-fluorophenyl)-1-[3-(4-Morpholinyl)propyl]imidazole

### a) 2-Aminopyrimidine-4-carboxaldehyde dimethyl acetal

Dimethylformamide dimethyl acetal (55 mL, 0.41 mol), and pyruvic aldehyde dimethyl acetal (50 mL, 0.41 mol) were combined and heated to 100° for 18 h. Methanol was removed in vacuo to afford an oil.

A solution of NaOH (18 g, 0.45 mol) in H₂O (50 mL) was added to guanidine HCl (43 g, 0.45 mol) in H₂O (100 mL), and the resulting solution was added to the above described oil. The resulting mixture was stirred at 23° for 48 h. Filtration afforded 25g (50%) of the title compound.

### b) 2-Aminopyrimidine-4-carboxaldehyde

The compund of the previous step (1.69 g, 10 mmol) and 3N HCl (7.3 mL, 22 mmol) were combined and heated to 48° for 14h, cooled, layered with EtOAc (50 mL) and neutralized by the addition of NaHCO₃ (2.1g, 25 mmol) in small portions. The aq phase was extracted with EtOAc (5 x 50 mL) and the extracts were dried (Na₂SO₄)and concentrated to afford 0.793 g (64%) of the title compound.

### c) 2-Aminopyrimidine-4-carboxaldehyde [3-(4-Morpholinyl)propyl]imine

The compound of the previous step and 4-(3-aminopropyl)morpholine were reacted by the procedure of example 1(c) to afford the title compound as a yellow oil.

### d) 5-(2-Aminopyrimidin-4-yl)-4-(4-fluorophenyl)-1-[3-(4-Morpholinyl)propyl]imidazole SB 216385

Following the procedure of example 1(d) except using the compound of the previous step as the imine afforded the title compound as a white solid. ¹H NMR (CD₃Cl) δ 8.15(d, J = 5.4 Hz, 1H), 7.62(s,1H), 7.46 (dd, 2H), 7.00(t, J = 8.6Hz, 2H), 6.50(d, J = 5.4 Hz, 1H), 5.09(brd.s, 2H), 4.34(t, J = 7.0 Hz, 2H), 3.69(m, 4H), 2.35(brd.s,4H), 2.24(t, J = 4.6 Hz, 2H), 1.84(m, 2H).

### Example 42

### 5-(2-Aminopyrimidin-4-yl)-4-(4-fluorophenyl)-1-(1-benzylpineridin-4-yl)imidazole a) 2-Aminopyrimidine-4-carboxaldehyde (1-benzylpiperidin-4-yl)imine

2-Aminopyrimidine-4-carboxaldehyde and 4-aminobenzylpiperidine were reacted by the procedure of example 1(c) to afford the title compound as a yellow oil.

### b) 5-(2-Aminopyrimidin-4-yl)-4-(4-fluorophenyl)-1-(1-benzylpiperidin-4-yl)imidazole

Following the procedure of example 1(d) except using the compound of the previous step as the imine afforded the title compound as a white solid (31% from the amine). mp = 227 - 229° (dec).

### Example 43

### 5-(2-Aminopyrimidin-4-yl)-4-(4-fluorophenyl)-1-(2-propyl)imidazole a) 2-Aminopyrimidine-4-carboxaldehyde (2-propyl)imine

2-Aminopyrimidine-4-carboxaldehyde and 2-propyl amine were reacted by the procedure of example 1(c) to afford the title compound as a yellow oil.

### b) 5-(2-Aminopyrimidin-4-yl)-4-(4-fluorophenyl)-1-(2-propyl)imidazole

Following the procedure of example 1(d) except using the compound of the previous step as the imine afforded the title compound as a white solid (32% from the 2-aminopyrimidine aldehyde). mp = 201 - 202°.

### Example 44

### 5-(2-Aminopyrimidin-4-yl)-4-(4-fluorophenyl)-1-(cyclopropylmethyl)imidazole a) 2-Aminopyrimidine-4-carboxaldehyde (cyclopropylmethyl)imine

2-Aminopyrimidine-4-carboxaldehyde and 2-cyclopropylmethyl amine were reacted by the procedure of example 1(c) to afford the title compound as a yellow oil.

### b) 5-(2-Aminopyrimidin-4-yl)-4-(4-fluorophenyl)-1-(cyclopropylmethyl)imidazole

Following the procedure of example 1(d) except using the compound of the previous step as the imine afforded the title compound as a white solid (38% from the 2-aminopyrimidine aldehyde). mp = 187 - 188°.

### Example 45

### 5-(2-Aminopyrimidin-4-yl)-4-(4-fluorophenyl)-1-(1-carboxyethyl-4-piperidinyl)imidazole

### a) 2-Aminopyrimidine-4-carboxaldehyde (1-carboxyethyl-4-piperidinyl)imine

2-Aminopyrimidine-4-carboxaldehyde and 1-carboxyethyl-4-aminopiperidine were reacted by the procedure of example 1(c) to afford the title compound as a yellow oil.

### b) 5-(2-Aminopyrimidin-4-yl)-4-(4-fluorophenyl)-1-(1-carboxyethyl-4-piperidinyl)imidazole

Following the procedure of example 1(d) except using the compound of the previous step as the imine afforded the title compound as a white solid (26% from the 2-aminopyrimidine aldehyde). mp = 216 - 218° (dec).

### Example 46

### 5-(2-Aminopyrimidin-4-yl)-4-(4-fluorophenyl)-1-(4-piperidinyl)imidazole

### a) 2-Aminopyrimidine-4-carboxaldehyde (1-t-butoxycarbonyl-4-aminopiperidinyl)imine

2-Aminopyrimidine-4-carboxaldehyde prepared in Example 41 and 1-t-butoxycarbonyl-4-aminopiperidine (Mach R. H., et.al., J. Med. Chem. **36**, p3707-3719, 1993) were reacted by the procedure of example 1(c) to afford the title compound as a yellow oil.

### b) 5-[4-(2-Amino)pyrimidinyl]-4-(4-fluorophenyl)-1-(1-t-butoxycarbonylpiperidin-4-yl)imidazole

Following the procedure of example 1(d) except using the compound of the previous step as the imine afforded the title compound as a white solid (27% from the 2-aminopyrimidine aldehyde).

### c) 5-[4-(2-Amino)pyrimidinyl]-4-(4-fluorophenyl)-1-(4-piperidinyl) imidazole SB-220025

The compound of the previous step was combined with 4N HCl in dioxane (5 mL), stirred 10 min, diluted wilth EtOAc and the liquid phase was decanted. The solid was twice washed with Et₂O (25 mL) and the liquid phase was decanted. Further trituration with EtOH (abs) and then Et₂O and drying in vacuo at 50° for 16 h afforded the title compound as the trihydrochloride (41%). mp = 265 - 275 (dec)

### Example 47

### 1-Methyl-4-phenyl-5-(4-pyridyl)imidazole

Following the procedure of example 48(b) except using benzonitrile the title compound as a white solid. mp = 161-162°.

### Example 48

### 1-Methyl-4-[3-(chlorophenyl)]-5-[4-pyridinyl]imidazole

### a) N-(4-Pyridinylmethyl)-N'-methylformamide

To a stirring, argon-purged solution of 4-picolyl chloride-HCl ( 15g, 91.4 mmol) and N-methylformamide (53.4 ml, 914 mmol) in 300 ml of THF at room temperature was added portionwise over a 20 minute period a suspension of 80% NaH (5.48g, 183mmol). The reaction was quenched 18h later by the addition of ice, partitioned between methylene chloride and water, washed with water and brine, dried over MgSO₄, and evaporated to dryness to afford a dark oil. Flash chromatography on silica gel provided 10.5g (76%) of the titled compounds a pale yellow oil. TLC; silica gel (9:1 CHCl/ MeOH) Rf = 0.54.

### b) 1-Methyl-4-[3-(chlorophenyl)1-5-[4-pyridinyllimidazole

To a stirred, argon-covered, -78° solution of lithium diisopropylamide (hereinafter LDA), (prepared from 11.2 ml of diisopropylamine in 150 ml of tetrahydrofuran (hereinafter THF) by the addition of 31.9 ml of 2.5M n-BuLi in hexanes) was added dropwise the product of the previous reaction (10g, 66.5 mmol) in 100 mL of THF. Stirring of the resultant reddish-brown solution was continued at -78° for 40 min at which point 3-chlorobenzonitrile (18.3g, 133 mmol) in 100 mL THF was added dropwise over 20 min. The reaction was allowed to warm to room temperature, stirred for 1h and heated to reflux for 12 h. The reaction was cooled and worked up in a manner similar to the previous reaction. Flash chromatography on silica gel provided 2.15g of an oil which was crystallized by dissolution with heating in 10 mL of ethyl acetate. Following crystallization, the solid was collected, washed, and dried (0.4 mm Hg) to afford 1.43g (8%) of the titled compound as a light tan solid. mp = 119-121°.

### Example 49

### 1-Methyl-4-(3-methylthiophenyl)-5-(4-pyridyl)imidazole

Following the procedure of example 48(b) except using the compound of example 13(b) as the aryl nitrile the title compound was obtained as a white solid. ES (+) MS m/e = 281 (MH⁺)

### Example 50

### 1-Methyl-4-(3-methylsulfinylphenyl)-5-(4-pyridyl)imidazole

Following the procedure of example 14 except using the compound of example 49 as the sulfide the title compound was obtained as a white solid. ES (+) MS m/e = 297 (MH⁺)

### Example 51

### (+/-)-4-(4-Fluorophenyl)-1-[3-(methylsulfinyl)propyl]-5-(4-pyridinyl)imidazole

Following the procedure of example 14 except using the compound from example 11 as the sulfide and quenching with saturated aq NH₄OH afforded the title compound as a white solid (0.87 g, 80%). mp = 122 - 123°.

### Example 52

### 4-(4-Fluorophenyl)-1-[(3-methylsulfonyl)propyl]-5-(4-pyridinyl)imidazole

The compound of Example 51 (0.5087 g, 1.48 mmol) was dissolved in methanol (8 ml) and cooled to 0°C. The addition of trifluoroacetic acid (0.12 ml) was followed by the dropwise addition of meta-chloroperoxybenzoic acid (0.23 g, 2.22 mmol) dissolved in CH₂Cl₂ (10 ml). After stirring for 1 h the solvents were evaporated. The residue was partitioned between H₂O and EtOAc and the aqueous phase was made basic by the addition of 2N NaOH. The organic phase was separated, dried (MgSO₄) and concentrated and the residue was pruified by flash chromatography (silica gel, 5% MeOH/CH₂Cl₂) to afford the title compound (0.37 g, 69%). mp = 146 - 147°.

### Example 53

### 1-(3-Phenoxypropyl)-4-(4-fluorophenyl)-5-(4-pyridinyl)imidazole

To a solution of the compound from example 2(b) (0.22 g, .70 mmol) in acetonitrile (10 ml) was added K₂CO₃ (0.19 g, 1.40 mmol) and phenol (0.10 g, 1.05 mmol). After stirring at 70°C for 24 h the reaction was diluted with H₂O. The organic phase was separated and concentrated and the residue was purified by flash chromatography (silica gel, 5% MeOH/CH₂Cl₂) followed by recrystalization in hexane to afford the title compound (0.02 g, 8%) as a white solid. mp = 95 - 96°.

### Example 54

### 1-[3-(Phenylthio)propyl]-4-(4-fluorophenyl)-5-(4-pyridinyl)imidazole

Following the procedure for example 3 except using thiophenol as the nucleophile, adding 2.2 eq K₂CO₃ and omitting the NaI. The cooled reaction was diluted with 10% NaOH and the product was extracted with ether. Flash chromatography was followed by recrystalization from hexane to afford the title compound (0.13 g, 53%) as white needles. mp = 98 - 99°.

### Example 55

### 1-[3-(4-Morpholinyl)propyl]-4-(4-fluorophenyl)-5-(4-quinolyl)imidazole

### a) Quinoyl-4-carboxaldehyde [3-(4-Morpholinyl)propyl]imine

Quinoyl-4-carboxaldehyde and 4-(3-aminopropyl)morpholine were reacted by the procedure of example 1(c) to afford the title compound as a yellow oil.

### b) 1-[3-(4-Morpholinyl)propyl]-4-(4-fluorophenyl)-5-(4-quinolyl)imidazole

Following the procedure of example 1(d) except using the compound of the previous step as the imine afforded the title compound as a white solid (48% from the amine). mp = 139.5 - 140.0.

### Example 56

### (+/-)-1-(3-Phenylsulfinylpropyl)-4-(4-fluorophenyl)-5-(4-pyridinyl)imidazole

Following the procedure of example 14 except using the compound from example 54 as the sulfide and quenching with saturated aq NH₄OH afforded the title compound as a white solid. mp = 146.5 - 148°.

### Example 57

### 1-(3-Ethoxypropyl)-4-(4-fluorophenyl)-5-(4-pyridinyl)imidazole

To a solution of the compound of example 2(b) (0.40 g, 1.26 mmol) in ethanol 25 ml) was added sodium ethoxide (0.8 ml, 21 wt% in ethanol). After refluxing 16 h the mixture was cooled, diluted with H₂O and extracted with EtOAc. Concentration of the solvent and purification by flash chromatography (silica gel, 5% MeOH/ CH₂Cl₂) afforded the title compound (0.05 g, 12 %). mp = 85 - 86°.

### Example 58

### 1-(3-Phenylsulfonylpropyl-4-(4-fluorophenyl)-5-(4-pyridinyl)imidazole

Following the procedure for example 52 except using the compound from example 56 as the sulfoxide and recrystalizing from hexane following the chromatography afforded the title compound as white solid. mp = 109 - 110°.

### Example 59

### 1-[3-(4-Morpholinyl)propyl]-4-(3-chlorophenyl)-5-(4-pyridyl)imidazole

### a) 3-chlorophenyl-tolylthiomethylisocyanide

Following the procedure of example 1(a,b) except using 3-chlorobenzaldehyde as the aldehyde component the title compound was prepared.

### b) 1-[3-(4-Morpholinyl)propyl]-4-(3-chlorophenyl)-5-(4-pyridyl)imidazole

Following the procedure of example 1(d) substituting the isocyanide prepared in the previous step the title compound was prepared. MS-DCI NH₃ = 383 [M+H].

### Example 60

### 1-[3-(4-Morpholinyl)propyl]-4-(3,4-dichlorophenyl)-5-(4-pyridyl)imidazole

Following the procedure of example 1(d) substituting the isocyanide prepared in Example 67(a) the title compound was prepared. mp = 106°.

### Example 61

### 4-[4-(4-Fluorophenyl)-1-[3-(4-morpholinyl)propyl]-5-(pyrimid-2-one-4-yl)imidazole a) 2-Methylthiopyrimidine-4-carboxaldehyde [3-(4-morpholinyl)propyl]imine

Following the procedure of example 1(c) except using 2-methylthiopyrimidine-4-carboxaldehyde [Bredereck H. et al. Chem. Ber. 1964, 3407] afforded the title compound as a yellow oil.b) 4-(4-Fluorophenyl)-1-[3-(4-morpholinyl)propyl]-5-(pyrimid-2-one-4-yl)imidazole

Concentrated aqueous ammonium hydroxide (2 mL) was added to 4-(4-fluorophenyl)-5-[2-(methylsulfinyl)-4-pyrimidinyl]-1-[3-(4-morpholinyl)propyl]-imidazole (0.14g, 0.37 mmol) [prepared in Example 63] and the reaction mixture was heated to 150°C for 18 h. After cooling to ambient temperature, the ammonium hydroxide was decanted. The residue was purified by flash chromatography eluting successively with 4% and 10% methanol in dichloromethane followed by successive elutions with mixtures of 90/10/1 and 70/30/3 chloroform/methanol/concentrated ammonium hydroxide. Trituration with ether afforded the title compound as an off-white solid (0.035 g, 24%). ESMS (*m*/*z*): 384 (M⁺ +H).

### Example 62

### 4-(4-Fluorophenyl)-5-[2-(methylthio)-4-pyrimidinyl]-1-[3-(4-morpholinyl)propyl]imidazole

Following the procedure of 1(d) except using 2-methylthiopyrimidine-4-carboxaldehyde [3-(4-morpholinyl)propyl]imine [prepared in Example 61(a)] afforded the title compound as a yelow oil. ¹H NMR (CDCl₃) δ 8.31(d, J = 7 Hz, 1H), 7.64(s,1H), 7.46 (dd, 2H), 7.05(t, J = 8 Hz, 2H), 6.81(d, J = 5 Hz, 1H), 4.42(t, J = 7.5 Hz, 2H), 3.71(t, J = 5Hz, 4H), 2.58(s, 3H), 2.37(brd. s, 4H), 2.27(t, J = 6 Hz, 2H), 1.85(m, 2H).

### Example 63

### 4-(4-Fluorophenyl)-5-[2-(methylsulfinyl)-4-pyrimidinyl]-1-[3-(4-morpholinyl)propyl]imidazole

A solution of K₂S₂O₈ (0.20 g, 0.73 mmol) in water (5 mL) was added to 4-(4-fluorophenyl)-5-[2-(methylthio)-4-pyrimidinyl]-1-[3-(4-morpholinyl)propyl]imidazole (0.20 g, 0.48 mmol) in glacial acetic acid (10 mL). After stirring at ambient temperature for 72 h, the reaction mixture was poured into water, neutralized with concentrated aqueous ammonium hydroxide and extracted four times with dichloromethane. The organic phases were combined and evaporated. The residue was purified by flash chromatography eluting successively with 1%, 2%, 4% and 10% methanol in dichloromethane to afford the title compound as a clear oil (0.15 g, 73%). ¹H NMR (CDCl₃) δ 8.57(d, J = 7 Hz, 1H), 7.77(s,1H), 7.47 (dd, 2H), 7.18(d, J = 5 Hz, 1H)7.09(t, J = 9 Hz, 2H), 4.56(m, 2H), 3.72(t, J = 5Hz, 4H), 3.00(s, 3H), 2.40(brd. s, 4H), 2.33(t, J = 8 Hz, 2H), 1.94(m, 2H).

### Example 64

### (E)-1-(1-Propenyl)-4-(4-fluorophenyl)-5-(4-pyridinyl)imidazole

### a) Pyridine-4-carboxaldehyde (2-propenyl)imine

Pyridine-4-carboxaldehyde and 2-propenyl amine were reacted by the procedure of example 1(c) to afford the title compound as a yellow oil.

### b) (E)-1-(1-Propenyl)-4-(4-fluorophenyl)-5-(4-pyridinyl)imidazole

Following the procedure of example 1(d) except using the compound of the previous step as the imine afforded a mixture of the title compound and 1-(2-Propenyl)-4-(4-fluorophenyl)-5-(4-pyridinyl)imidazole. Chromatography of the mixture with 0 - 50% EtOAc in hexanes afforded the title compound (43%). mp = 173.5 - 174.0

### Example 65

### 1-(2-Propenyl)-4-(4-fluorophenyl)-5-(4-pyridinyl)imidazole

Furthur chromatography of the mixture from example 64(b) afforded the title compound (54%). mp = 116.0 - 117.0

### Example 66

### 5-[(2-N,N-Dimethylamino)pyrimidin-4-yl]-4-(4-fluorophenyl)-1-[3-(4-morpholinyl)propyl]imidazole

Following the procedure of example 61(b) except using aqueous dimethylamine afforded the title compound as a yellow glass. ESMS (*m*/*z*): 411 (M⁺ +H).

### Example 67

### 1-[3-(4-Morpholinyl)propyl]-5-(4-pyridinyl)-4-[4-(trifluoromethyl)phenyl]imidazolea) 4-trifluoromethylphenyl-tolylthiomethylisocyanide

Following the procedure of example 1(a,b) except using 4-trifluoromethylbenzaldehyde as the aldehyde component the title compound was prepared.

### b) 1-[3-(4-Morpholinyl)propyll-5-(4-pyridinyl)-4-[4-(trifluoromethyl)-phenyl]imidazole

The imine prepared in Example 1(c) was reacted with the isocyanide prepared in the previous step using the procedure of example 1(d) to prepare the title compound was prepared. mp 133°.

### Example 68

### 1-[3-(4-Morpholinyl)propyl]-5-(4-pyridinyl)-4-[3-(trifluoromethyl)phenyl]imidazole

### a) 3-trifluoromethylphenyl-tolylthiomethylisocyanide

Following the procedure of example 1(a,b) except using 3-trifluoromethylbenzaldehyde as the aldehyde component the title compound was prepared.

### b) 1-[3-(4-Morpholinyl)propyl]-5-(4-pyridinyl)-4-[4-(trifluoromethyl)-phenyl]imidazole

The imine prepared in Example 1(c) was reacted with the isocyanide prepared in the previous step using the procedure of example 1(d) to prepare the title compound was prepared. ESMS = 417 [M+H]

### Example 69

### 1-(Cyclopropylmethyl)-4-(3,4-dichlorophenyl)-5-(4-pyridinyl)imidazole a) 3,4-dichlorophenyl-tolylthiomethylisocyanide

Following the procedure of example 1(a,b) except using 3,4-dichlorobenzaldehyde as the aldehyde component the title compound was prepared.

### b) 1-(Cyclopropylmethyl)-4-(3,4-dichlorophenyl)-5-(4-pyridinyl)imidazole

Following the procedure of example 1(d) substituting the imine prepared in Example 27(a) and the isocyanide prepared in the previous step the title compound was prepared. mp = 145.5°.

### Example 70

### 1-(Cyclopropylmethyl)-4-(3-trifluoromethylphenyl)-5-(4-pyridinyl)imidazole

Following the procedure of Example 1(d) substituting the imine prepared in Example 27(a) and the isocyanide prepared Example 68(a) the title compound was prepared. mp = 105.5°.

### Example 71

### 1-(Cyclopropylmethyl)-4-(4-fluorophenyl)-5-(2-methylpyrid-4-yl)imidazole

### a) 2-Methylpyridine-4-carboxaldehyde (cyclopropylmethyl)imine

Reaction of 4-formyl-2-methylpyridine [prepared in Example 33(a)] and cyclopropylmethyl amine by the procedure of example 1(c) affords the title compound as a yellow oil.

### b) 1-(Cyclopropylmethyl)-4-(4-fluorophenyl)-5-(2-methylpyrid-4-yl)imidazole

Following the procedure of example 1(d) except using the compound of the previous step as the imine afforded the title compound was as a white solid (62% from the 2-aminopyrimidine aldehyde). mp = 141.0 - 141.5°.

### Example 72

### 1-[3-(4-Morpholinyl)propyl]-5-(4-pyridinyl)-4-(3,5-bistrifluoromethylphenyl)-imidazole

### a) 3,5-bistrifluoromethylphenyl-tolylthiomethylisocyanide

Following the procedure of example 1(a,b) except using 3,5-bistrifluoromethylbenzaldehyde as the aldehyde component the title compound was prepared.

### b) 1-[3-(4-Morpholinyl)propyll-5-(4-pyridinyl)-4-(3,5-bistrifluoromethylphenyl)imidazole

Following the procedure of example 1(d) substituting the imine prepared in Example 1(c) and the isocyanide prepared in the previous step the title compound was prepared. mp = 136.5-137.5°.

### Example 73

### 5-[4-(2-Aminopyrimidinyl)]-4-(4-fluorophenyl)-1-(2-carboxy-2,2-dimethylethyl)-imidazole a) 2-Aminopyrimidine-4-carboxaldehyde (ethyl 3-amino-2,2-dimethylpropionate)imine

2-Aminopyrimidine-4-carboxaldehyde and ethyl 3-amino-2,2-dimethylpropionate, were reacted by the procedure of example 1(c) to afford the title compound as a yellow oil.

### b) 5-[4-(2-Aminopyrimidinyl)]-4-(4-fluorophenyl)-1-(2-carboxyethyl-2,2-dimethylpropyl)imidazole

Following the procedure of example 1(d) except using the compound of the previous step as the imine afforded the title compound as a white solid (11% from the amine).

### c) 5-[4-(2-Aminopyrimidinyl)1-4-(4-fluorophenyl)-1-(2-carboxy-2,2-dimethylethyl)imidazole lithium salt

The compound of example 73(c) was hydrolysed by the procedure of example 37 to afford the title compound as the lithium salt; (67%). ES (+) MS m/e = 356.

### Example 74

### 1-(1-Formyl-4-piperidinyl)-4-(4-fluorophenyl)-5-(4-pyridinyl)imidazole

1-(1-Benzylpiperidin-4-yl)-4-(4-fluorophenyl)-5-(4-pyridyl)imidazole (100 mg;prepared in Example 40) was dissolved in 10% formic acid/methanol under argon and palladium black (100 mg) mixed in 10% formic acid/methanol was added. The reaction was stirred under argon at room temperature for sixteen hours. The reaction mixture was evaporated and the residue mixed in H₂O/ethyl acetate and the pH taken to 10. The layers were separated and the aqueous phase extracted with ethyl acetate. The combined organic layers were evaporated and the residue was flash chromatographed (silica gel/methylene chloride/methanol) to yield the title compound, an off-white solid. ES (+) MS m/e = 351 (MH⁺)

### Example 75

### 5-(2-Amino-4-pyrimidinyl)-4-(4-fluorophenyl)-1-(1-methyl-4-piperidinyl)imidazole

### a) 4-Amino-1-methylpiperidine

1-Methylpiperidin-4-one (4.22 g, 37 mmol) and an ice cold soln of 1N HCl in Et₂O (37 mL, 37 mmol) were combined. Trituration followed by evaporation of the Et₂O at 23° under a stream of argon afforded the hydrochloride. MeOH (114 mL), anhydrous NH₄OAc (28.7, 373 mmol) and 3A molecular sieves were added. Stirred 10 min and then NaCNBH₃ (2.33 g, 37 mmol) was added, and the mixture was stirred for 1 h. Acidified to < pH 1 with concentrated HCl and washed with Et₂O. The resulting mixture was made basic with 50% aq NaOH and extracted with EtOAc, dried (K₂CO₃), and distilled (bp = 55 - 60°, 15 mm) to afford 3.88 g (88%) of the title compound.

### b) 2-Aminopyrimidine-4-carboxaldehyde (1-methylpiperidin-4-yl)imine

2-Aminopyrimidine-4-carboxaldehyde and the compound of the previous step were reacted by the procedure of example 1(c) to afford the title compound as a yellow oil.

### b) 5-(2-Aminopyrimidin-4-yl)-4-(4-fluorophenyl)-1-(1-benzylpiperidin-4-yl)imidazole

Following the procedure of example 1(d) except using the compound of the previous step as the imine afforded the title compound after purification by chromatography on silica with 0 - 10% MeOH and 0 - 1% Et₃N in CH₂Cl₂ followed by fractional preciptation from MeOH with Et₂O; as a yellow solid (20% from the amine). mp = 235 - 237° (dec.)

### Example 76

### 1-(2,2-Dimethyl-3-morpholin-4-yl)propyl-4-(4-fluorophenyl)-5-(2-Amino-4-pyrimidinyl)imidazole

### a) N-(1-Amino-2,2-dimethylpropyl)morpholine

2,2-dimethyl-3-N-morpholinyl propionaldehyde (Cheney,L.L. J. Amer. Chem. Soc. 1951, 73, p685 - 686; 855 mg, 5.0 mmol) was dissolved in Et₂O (2 mL) and 1 N HCl in Et₂O (5 mL, 5 mmol) was added. Stirred 5 min and the Et₂O was evaporated in a stream of Ar. The solid was dissolved in anhydrous MeOH (15 mL) followed by anhydrous NH₄OAc (3.85 g, 50 mmol), and 3A molecular sieves. Stirred 5 min and then NaCNBH₃ (0.314 g, 4.0 mmoL) was added. Stirred 45 min and concd HCl was added til the reaction mixture was < pH 1. The MeOH was removed in vacuo and the residual mixture was dissolved in H₂O (15 mL) and extracted with Et₂O (25 mL). The aq phase was layered with another portion of Et₂O and made basic by addition of 50% aq NaOH til > pH 10. Extraction with Et₂O (3 x 40 mL), drying (K₂CO₃) and concentration afforded the title compound (86%).

### b) 2-Aminopyrimidine-4-carboxaldehyde [3-(4-Morpholinyl)-2,2-dimethylpropyl]imine

2-Aminopyrimidine-4-carboxaldehyde and the product of the previous step were reacted by the procedure of example 1(c) to afford the title compound as a yellow oil.

### c) 1-(2,2-Dimethyl-3-morpholin-4-yl)propyl-4-(4-fluorophenyl)-5-(2-Amino-4-pyrimidinyl)imidazole

Following the procedure of example 1(d) except using the compound of the previous step as the imine afforded the title compound as a white solid (16% from the amine). mp = 242 - 245° (dec.).

### Example 77

### 4-(4-Fluorophenyl)-5-(4-pyridyl)-1-(2-acetoxyethyl)imidazole

500 mg of 4-(4-Fluorophenyl)-5-(4-pyridyl)imidazole was dried at 50• C overnight in vacuo and added to a flask containing 20 ml of dried (sieve) dimethyl formamide (hereinafter DMF) and treated with NaH (at 0 •C), then stirred at room temperature, and dropwise with 2-acetoxy ethylbromide. After three days, the mixture was poured into ice water, extracted into methyoene chloride, the organic phase washed with water, dried over sodium sulfate and stripped in vacuo. Flashed the residue on silica using CH₂Cl₂-acetone (85:15) and eluting with increasing CH₃OH from 0 to 10%. Two major product fractions were obtained, the pure cuts combined to give a slower eluting fraction and a faster eluting isomer. These isomers were stripped and recrystallized from EtOAc-hexane to give the minor isomer (slower moving) and the fast, major isomer (the titled compound). NMR (250mHz, CDCl₃) shows CH₂CH₂ as singlet at δ 4.1 ppm, very clean, H-ortho to F, triple t at 6.9 ppm. Cal'd C:66.60, H:4.86, N:12.92; Found C:67.10, 67.03 H:5.07, 4.94 N:13.08, 13.09. IR (nujol mull) shows 1740 cm⁻¹ (sharp, ester).

### METHODS OF TREATMENT

The compounds of Formula (I) or a pharmaceutically acceptable salt thereof can be used in the manufacture of a medicament for the prophylactic or therapeutic treatment of any disease state in a human, or other mammal, which is exacerbated or caused by excessive or unregulated cytokine production by such mammal's cell, such as but not limited to monocytes and/or macrophages.

Compounds of Formula (I) are capable of inhibiting proinflammatory cytokines, such as IL-1, IL-6, IL-8 and TNF and are therefore of use in therapy. IL-1, IL-6, IL-8 and TNF affect a wide variety of cells and tissues and these cytokines, as well as other leukocyte-derived cytokines, are important and critical inflammatory mediators of a wide variety of disease states and conditions. The inhibition of these pro-inflammatory cytokines is of benefit in controlling, reducing and alleviating many of these disease states.

Accordingly, the present invention provides a method of treating a cytokine-mediated disease which comprises administering an effective cytokine-interfering amount of a compound of Formula (I) or a pharmaceutically acceptable salt thereof.

In particular, compounds of Formula (I) or a pharmaceutically acceptable salt thereof are of use in the prophylaxis or therapy of any disease state in a human, or other mammal, which is exacerbated by or caused by excessive or unregulated IL-1, IL-8 or TNF production by such mammal's cell, such as, but not limited to, monocytes and/or macrophages.

Accordingly, in another aspect, this invention relates to a method of inhibiting the production of IL-1 in a mammal in need thereof which comprises administering to said mammal an effective amount of a compound of Formula (I) or a pharmaceutically acceptable salt thereof.

There are many disease states in which excessive or unregulated IL-1 production is implicated in exacerbating and/or causing the disease. These include rheumatoid arthritis, osteoarthritis, endotoxemia and/or toxic shock syndrome, other acute or chronic inflammatory disease states such as the inflammatory reaction induced by endotoxin or inflammatory bowel disease, tuberculosis, atherosclerosis, muscle degeneration, multiple sclerosis, cachexia, bone resorption, psoriatic arthritis, Reiter's syndrome, rheumatoid arthritis, gout, traumatic arthritis, rubella arthritis and acute synovitis. Recent evidence also links IL-1 activity to diabetes, pancreatic β cells and Alzheimer's disease.

In a further aspect, this invention relates to a method of inhibiting the production of TNF in a mammal in need thereof which comprises administering to said mammal an effective amount of a compound of Formula (I) or a pharmaceutically acceptable salt thereof.

Excessive or unregulated TNF production has been implicated in mediating or exacerbating a number of diseases including rheumatoid arthritis, rheumatoid spondylitis, osteoarthritis, gouty arthritis and other arthritic conditions, sepsis, septic shock, endotoxic shock, gram negative sepsis, toxic shock syndrome, adult respiratory distress syndrome, cerebral malaria, chronic pulmonary inflammatory disease, silicosis, pulmonary sarcoisosis, bone resorption diseases, such as osteoporosis, reperfusion injury, graft vs. host reaction, allograft rejections, fever and myalgias due to infection, such as influenza, cachexia secondary to infection or malignancy, cachexia secondary to acquired immune deficiency syndrome (AIDS), AIDS, ARC (AIDS related complex), keloid formation, scar tissue formation, Crohn's disease, ulcerative colitis and pyresis.

Compounds of Formula (I) are also useful in the treatment of viral infections, where such viruses are sensitive to upregulation by TNF or will elicit TNF production *in vivo.* The viruses contemplated for treatment herein are those that produce TNF as a result of infection, or those which are sensitive to inhibition, such as by decreased replication, directly or indirectly, by the TNF inhibiting-compounds of Formula (1). Such viruses include, but are not limited to HIV-1, HIV-2 and HIV-3, Cytomegalovirus (CMV), Influenza, adenovirus and the Herpes group of viruses, such as but not limited to, Herpes Zoster and Herpes Simplex. Accordingly, in a further aspect, this invention relates to a method of treating a mammal afflicted with a human immunodeficiency virus (HIV) which comprises administering to such mammal an effective TNF inhibiting amount of a compound of Formula (I) or a pharmaceutically acceptable salt thereof.

Compounds of Formula (I) may also be used in association with the veterinary treatment of mammals, other than in humans, in need of inhibition of TNF production. TNF mediated diseases for treatment, therapeutically or prophylactically, in animals include disease states such as those noted above, but in particular viral infections. Examples of such viruses include, but are not limited to, lentivirus infections such as, equine infectious anaemia virus, caprine arthritis virus, visna virus, or maedi virus or retrovirus infections, such as but not limited to feline immunodeficiency virus (FIV), bovine immunodeficiency virus, or canine immunodeficiency virus or other retroviral infections.

The compounds of Formula (I) may also be used topically in the treatment or prophylaxis of topical disease states mediated by or exacerbated by excessive cytokine production, such as by IL-1 or TNF respectively, such as inflamed joints, eczema, psoriasis and other inflammatory skin conditions such as sunburn; inflammatory eye conditions including conjunctivitis; pyresis, pain and other conditions associated with inflammation.

Compounds of Formula (I) have also been shown to inhibit the production of IL-8 (Interleukin-8, NAP). Accordingly, in a further aspect, this invention relates to a method of inhibiting the production of IL-8 in a mammal in need thereof which comprises administering to said mammal an effective amount of a compound of Formula (I) or a pharmaceutically acceptable salt thereof.

There are many disease states in which excessive or unregulated IL-8 production is implicated in exacerbating and/or causing the disease. These diseases are characterized by massive neutrophil infiltration such as, psoriasis, inflammatory bowel disease, asthma, cardiac and renal reperfusion injury, adult respiratory distress syndrome, thrombosis and glomerulonephritis. All of these diseases are associated with increased IL-8 production which is responsible for the chemotaxis of neutrophils into the inflammatory site. In contrast to other inflammatory cytokines (IL-1, TNF, and IL-6), IL-8 has the unique property of promoting neutrophil chemotaxis and activation. Therefore, the inhibition of IL-8 production would lead to a direct reduction in the neutrophil infiltration.

The compounds of Formula (I) are administered in an amount sufficient to inhibit cytokine, in particular IL-1, IL-6, IL-8 or TNF, production such that it is regulated down to normal levels, or in some case to subnormal levels, so as to ameliorate or prevent the disease state. Abnormal levels of IL-1, IL-6, IL-8 or TNF, for instance in the context of the present invention, constitute: (i) levels of free (not cell bound) IL-1, IL-6, IL-8 or TNF greater than or equal to 1 picogram per ml; (ii) any cell associated IL-1, IL-6, IL-8 or TNF; or (iii) the presence of IL-1, IL-6, IL-8 or TNF mRNA above basal levels in cells or tissues in which IL-1, IL-6, IL-8 or TNF, respectively, is produced.

The discovery that the compounds of Formula (I) are inhibitors of cytokines, specifically IL-1, IL-6, IL-8 and TNF is based upon the effects of the compounds of Formulas (I) on the production of the IL-1, IL-8 and TNF in *in vitro* assays which are described herein.

As used herein, the term "inhibiting the production of IL-1 (IL-6, IL-8 or TNF)" refers to:
a) a decrease of excessive *in vivo* levels of the cytokine (IL-1, IL-6, IL-8 or TNF) in a human to normal or sub-normal levels by inhibition of the *in vivo* release of the cytokine by all cells, including but not limited to monocytes or macrophages;
b) a down regulation, at the genomic level, of excessive *in vivo* levels of the cytokine (IL-1, IL-6, IL-8 or TNF) in a human to normal or sub-normal levels;
c) a down regulation, by inhibition of the direct synthesis of the cytokine (IL-1, IL-6, IL-8 or TNF) as a postranslational event; or
d) a down regulation, at the translational level, of excessive *in vivo* levels of the cytokine (IL-1, IL-6, IL-8 or TNF) in a human to normal or sub-normal levels.

As used herein, the term "TNF mediated disease or disease state" refers to any and all disease states in which TNF plays a role, either by production of TNF itself, or by TNF causing another monokine to be released, such as but not limited to IL-1, IL-6 or IL-8. A disease state in which, for instance, IL-1 is a major component, and whose production or action, is exacerbated or secreted in response to TNF, would therefore be considered a disease stated mediated by TNF.

As used herein, the term "cytokine" refers to any secreted polypeptide that affects the functions of cells and is a molecule which modulates interactions between cells in the immune, inflammatory or hematopoietic response. A cytokine includes, but is not limited to, monokines and lymphokines, regardless of which cells produce them. For instance, a monokine is generally referred to as being produced and secreted by a mononuclear cell, such as a macrophage and/or monocyte. Many other cells however also produce monokines, such as natural killer cells, fibroblasts, basophils, neutrophils, endothelial cells, brain astrocytes, bone marrow stromal cells, epideral keratinocytes and B-lymphocytes. Lymphokines are generally referred to as being produced by lymphocyte cells. Examples of cytokines include, but are not limited to, Interleukin-1 (IL-1), Interleukin-6 (IL-6), Interleukin-8 (IL-8), Tumor Necrosis Factor-alpha (TNF-α) and Tumor Necrosis Factor beta (TNF-β).

As used herein, the term "cytokine interfering" or "cytokine suppressive amount" refers to an effective amount of a compound of Formula (I) which will cause a decrease in the *in vivo* levels of the cytokine to normal or sub-normal levels, when given to a patient for the prophylaxis or treatment of a disease state which is exacerbated by, or caused by, excessive or unregulated cytokine production.

As used herein, the cytokine referred to in the phrase "inhibition of a cytokine, for use in the treatment of a HIV-infected human" is a cytokine which is implicated in (a) the initiation and/or maintenance of T cell activation and/or activated T cell-mediated HIV gene expression and/or replication and/or (b) any cytokine-mediated disease associated problem such as cachexia or muscle degeneration.

As TNF-β (also known as lymphotoxin) has close structural homology with TNF-α (also known as cachectin) and since each induces similar biologic responses and binds to the same cellular receptor, both TNF-a and TNF-β are inhibited by the compounds of the present invention and thus are herein referred to collectively as "TNF" unless specifically delineated otherwise.

In order to use a compound of Formula (I) or a pharmaceutically acceptable salt thereof in therapy, it will normally be Formulated into a pharmaceutical composition in accordance with standard pharmaceutical practice. This invention, therefore, also relates to a pharmaceutical composition comprising an effective, non-toxic amount of a compound of Formula (I) and a pharmaceutically acceptable carrier or diluent.

Compounds of Formula (I), pharmaceutically acceptable salts thereof and pharmaceutical compositions incorporating such may conveniently be administered by any of the routes conventionally used for drug administration, for instance, orally, topically, parenterally or by inhalation. The compounds of Formula (I) may be administered in conventional dosage forms prepared by combining a compound of Formula (I) with standard pharmaceutical carriers according to conventional procedures. The compounds of Formula (I) may also be administered in conventional dosages in combination with a known, second therapeutically active compound. These procedures may involve mixing, granulating and compressing or dissolving the ingredients as appropriate to the desired preparation. It will be appreciated that the form and character of the pharmaceutically acceptable character or diluent is dictated by the amount of active ingredient with which it is to be combined, the route of administration and other well-known variables. The carrier(s) must be "acceptable" in the sense of being compatible with the other ingredients of the Formulation and not deleterious to the recipient thereof.

The pharmaceutical carrier employed may be, for example, either a solid or liquid. Exemplary of solid carriers are lactose, terra alba, sucrose, talc, gelatin, agar, pectin, acacia, magnesium stearate, stearic acid and the like. Exemplary of liquid carriers are syrup, peanut oil, olive oil, water and the like. Similarly, the carrier or diluent may include time delay material well known to the art, such as glyceryl monostearate or glyceryl distearate alone or with a wax.

A wide variety of pharmaceutical forms can be employed. Thus, if a solid carrier is used, the preparation can be tableted, placed in a hard gelatin capsule in powder or pellet form or in the form of a troche or lozenge. The amount of solid carrier will vary widely but preferably will be from about 25mg. to about 1g. When a liquid carrier is used, the preparation will be in the form of a syrup, emulsion, soft gelatin capsule, sterile injectable liquid such as an ampule or nonaqueous liquid suspension.

Compounds of Formula (I) may be administered topically, that is by non-systemic administration. This includes the application of a compound of Formula (I) externally to the epidermis or the buccal cavity and the instillation of such a compound into the ear, eye and nose, such that the compound does not significantly enter the blood stream. In contrast, systemic administration refers to oral, intravenous, intraperitoneal and intramuscular administration.

Formulations suitable for topical administration include liquid or semi-liquid preparations suitable for penetration through the skin to the site of inflammation such as liniments, lotions, creams, ointments or pastes, and drops suitable for administration to the eye, ear or nose. The active ingredient may comprise, for topical administration, from 0.001% to 10% w/w, for instance from 1% to 2% by weight of the Formulation. It may however comprise as much as 10% w/w but preferably will comprise less than 5% w/w, more preferably from 0.1% to 1% w/w of the Formulation.

Lotions according to the present invention include those suitable for application to the skin or eye. An eye lotion may comprise a sterile aqueous solution optionally containing a bactericide and may be prepared by methods similar to those for the preparation of drops. Lotions or liniments for application to the skin may also include an agent to hasten drying and to cool the skin, such as an alcohol or acetone, and/or a moisturizer such as glycerol or an oil such as castor oil or arachis oil.

Creams, ointments or pastes according to the present invention are semi-solid Formulations of the active ingredient for external application. They may be made by mixing the active ingredient in finely-divided or powdered form, alone or in solution or suspension in an aqueous or non-aqueous fluid, with the aid of suitable machinery, with a greasy or non-greasy base. The base may comprise hydrocarbons such as hard, soft or liquid paraffin, glycerol, beeswax, a metallic soap; a mucilage; an oil of natural origin such as almond, corn, arachis, castor or olive oil; wool fat or its derivatives or a fatty acid such as steric or oleic acid together with an alcohol such as propylene glycol or a macrogel. The Formulation may incorporate any suitable surface active agent such as an anionic, cationic or non-ionic surfactant such as a sorbitan ester or a polyoxyethylene derivative thereof. Suspending agents such as natural gums, cellulose derivatives or inorganic materials such as silicaceous silicas, and other ingredients such as lanolin, may also be included.

Drops according to the present invention may comprise sterile aqueous or oily solutions or suspensions and may be prepared by dissolving the active ingredient in a suitable aqueous solution of a bactericidal and/or fungicidal agent and/or any other suitable preservative, and preferably including a surface active agent. The resulting solution may then be clarified by filtration, transferred to a suitable container which is then sealed and sterilized by autoclaving or maintaining at 98-100• C. for half an hour. Alternatively, the solution may be sterilized by filtration and transferred to the container by an aseptic technique. Examples of bactericidal and fungicidal agents suitable for inclusion in the drops are phenylmercuric nitrate or acetate (0.002%), benzalkonium chloride (0.01%) and chlorhexidine acetate (0.01%). Suitable solvents for the preparation of an oily solution include glycerol, diluted alcohol and propylene glycol.

Compounds of formula (I) may be administered parenterally, that is by intravenous, intramuscular, subcutaneous intranasal, intrarectal, intravaginal or intraperitoneal administration. The subcutaneous and intramuscular forms of parenteral administration are generally preferred. Appropriate dosage forms for such administration may be prepared by conventional techniques. Compounds of Formula (I) may also be administered by inhalation, that is by intranasal and oral inhalation administration. Appropriate dosage forms for such administration, such as an aerosol Formulation or a metered dose inhaler, may be prepared by conventional techniques.

For all methods of use disclosed herein for the compounds of Formula (I), the daily oral dosage regimen will preferably be from about 0.1 to about 80 mg/kg of total body weight, preferably from about 0.2 to 30 mg/kg, more preferably from about 0.5 mg to 15mg. The daily parenteral dosage regimen about 0.1 to about 80 mg/kg of total body weight, preferably from about 0.2 to about 30 mg/kg, and more preferably from about 0.5 mg to 15mg/kg. The daily topical dosage regimen will preferably be from 0.1 mg to 150 mg, administered one to four, preferably two or three times daily. The daily inhalation dosage regimen will preferably be from about 0.01 mg/kg to about 1 mg/kg per day. It will also be recognized by one of skill in the art that the optimal quantity and spacing of individual dosages of a compound of Formula (I) or a pharmaceutically acceptable salt thereof will be determined by the nature and extent of the condition being treated, the form, route and site of administration, and the particular patient being treated, and that such optimums can be determined by conventional techniques. It will also be appreciated by one of skill in the art that the optimal course of treatment, i.e., the number of doses of a compound of Formula (I) or a pharmaceutically acceptable salt thereof given per day for a defined number of days, can be ascertained by those skilled in the art using conventional course of treatment determination tests.

The invention will now be described by reference to the following biological examples which are merely illustrative and are not to be construed as a limitation of the scope of the present invention.

### BIOLOGICAL EXAMPLES

The cytokine-inhibiting effects of compounds of the present invention were determined by the following *in vitro* assays:

### Interleukin - 1 (IL-1)

Human peripheral blood monocytes were isolated and purified from either fresh blood preparations from volunteer donors, or from blood bank buffy coats, according to the procedure of Colotta *et al*, J Immunol, **132,** 936 (1984). These monocytes (1x10⁶) were plated in 24-well plates at a concentration of 1-2 million/ml per well. The cells were allowed to adhere for 2 hours, after which time non-adherent cells were removed by gentle washing. Test compounds were then added to the cells for 1h before the addition of lipopolysaccharide (50 ng/ml), and the cultures were incubated at 37°C for an additional 24h. At the end of this period, culture super-natants were removed and clarified of cells and all debris. Culture supernatants were then immediately assayed for IL-1 biological activity, either by the method of Simon *et al*., J. Immunol. Methods, **84,** 85, (1985) (based on ability of IL-1 to stimulate a Interleukin 2 producing cell line (EL-4) to secrete IL-2, in concert with A23187 ionophore) or the method of Lee *et al*., J. ImmunoTherapy, **6**(1), 1-12 (1990) (ELISA assay). The compounds of Formula (I), as evidenced by Examples 1 to 24 were shown to be inhibitors of *in vitro* IL-1 produced by human monocytes.

### Tumour Necrosis Factor (TNF):

Human peripheral blood monocytes were isolated and purified from either blood bank buffy coats or plateletpheresis residues, according to the procedure of Colotta, R. *et al*., J Immunol, **132**(2), 936 (1984). The monocytes were plated at a density of 1x10⁶ cells/ml medium/well in 24-well multi-dishes. The cells were allowed to adhere for 1 hour after which time the supernatant was aspirated and fresh medium (1ml, RPMI-1640, Whitaker Biomedical Products, Whitaker, CA) containing 1% fetal calf serum plus penicillin and streptomycin (10 units/ml) added. The cells were incubated for 45 minutes in the presence or absence of a test compound at 1nM-10mM dose ranges (compounds were solubilized in dimethyl sulfoxide/ethanol, such that the final solvent concentration in the culture medium was 0.5% dimethyl sulfoxide/0.5% ethanol). Bacterial lipopoly-saccharide (*E*. *coli* 055:B5 [LPS] from Sigma Chemicals Co.) was then added (100 ng/ml in 10 ml phosphate buffered saline) and cultures incubated for 16-18 hours at 37°C in a 5% CO₂ incubator. At the end of the incubation period, culture supernatants were removed from the cells, centrifuged at 3000 rpm to remove cell debris. The supernatant was then assayed for TNF activity using either a radio-immuno or an ELISA assay, as described in WO 92/10190 and by Becker *et al*., J Immunol, 1991, **147,** 4307. The compounds of Formula (I), as evidenced by Examples 1 to 24 were shown to be inhibitors of *in vitro* TNF produced by human monocytes.

IL-1 and TNF inhibitory activity does not seem to correlate with the property of the compounds of Formula (I) in mediating arachidonic acid metabolism inhibition. Further the ability to inhibit production of prostaglandin and/or leukotriene synthesis, by nonsteroidal anti-inflammatory drugs with potent cyclooxygenase and/or lipoxygenase inhibitory activity does not mean that the compound will necessarily also inhibit TNF or IL-1 production, at non-toxic doses.

### Interleukin -8 (IL-8):

Primary human umbilical cord endothelial cells (HUVEC) (Cell Systems, Kirland, Wa) are maintained in culture medium supplemented with 15% fetal bovine serum and 1% CS-HBGF consisting of aFGF and heparin. The cells are then diluted 20-fold before being plated (250µl) into gelating coated 96-well plates. Prior to use, culture medium are replaced with fresh medium (200µl). Buffer or test compound (25µl, at concentrations between 1 and 10µM) is then added to each well in quadruplicate wells and the plates incubated for 6h in a humidified incubator at 37°C in an atmosphere of 5% CO₂. At the end of the incubation period, supernatant is removed and assayed for IL-8 concentration using an IL-8 ELISA kit obtained from R&D Systems (Minneapolis, MN). All data is presented as mean value (ng/ml) of multiple samples based on the standard curve. IC₅₀'s where appropriate are generated by nonlinear regression analysis.

### Cytokine Specific Binding Protein Assay

A radiocompetitive binding assay was developed to provide a highly reproducible primary screen for structure-activity studies. This assay provides many advantages over the conventional bioassays which utilize freshly isolated human monocytes as a source of cytokines and ELISA assays to quantify them. Besides being a much more facile assay, the binding assay has been extensively validated to highly correlate with the results of the bioassay. A specific and reproducible CSAID™ cytokine inhibitor binding assay was developed using soluble cystosolic fraction from THP.1 cells and a radiolabeled compound. For instance, a suitable radiolabeled compound of this CSAID ™ cytokine inhibitor class is 4-(Fluorophenyl)-2-(4-hydroxyphenyl-3,5-t₂)-5-(4-pyridyl)imidazole. In brief, the THP.1 cytosol was routinely prepared from cell lysate obtained by nitrogen cavitation followed by a 10 K x g low speed and a 100 K x g high speed centrifugation, the supernatant of which was designated as the cytosolic fraction. THP.1 cytosol was incubated with appropriately diluted radioligand at room temperature for a pre-determined time to allow the binding to achieve equilibrium. The sample was added to a G-10 column and eluted with 20 mm TRN, 50mMb - mercaptoethanol, NaN_{3.} The fraction encompassing the void volume was collected and the radioactivity was assessed by liquid scintillation counting. This was determined to reflect bound radioligand since the radioactive signal was abrogated by the presence of excess cold ligand in the incubation mixture or when there was no cytosolic fraction present. Compounds of Formula (I) at various doses were added to the binding assay to achieve inhibition of binding of the radiolabel. IC₅₀s as well as Ki values were determined by regression analysis and scatchard plot analysis respectively. There is generally excellent correlation between the IC₅₀ of compounds tested in both the binding assay and the bioassay and can be used interchangeably in many cases.

Patent Application USSN 08/123175 Lee et al., filed September 1993 whose disclosure is incorporated by reference herein in its entirey describes the above noted method for screening drugs to identify compounds which interact with and bind to the cytokine specific binding protein (hereinafter CSBP). However, for purposes herein the binding protein may be in isolated form in solution, or in immobilized form, or may be genetically engineered to be expressed on the surface of recombinant host cells such as in phage display system or as fusion proteins. Alternatively, whole cells or cytosolic fractions comprising the CSBP may be employed in the creening protocol. Regardless of the form of the binding protein, a plurality of compounds are contacted with the binding protein under conditions sufficient to form a compound/ binding protein complex and compound capable of forming, enhancing or interfering with said complexes are detected.

More specifically, the Binding Assay is performed as follows:
MATERIALS:
Incubation buffer: 20 mM Tris, 1 mM MgCl₂, 20 mM Hepes, 0.02% NaN₃, store at 4°C. Elution buffer: 20 mM Tris, 50 mM 2-mercaptoethanol, NaN₃, store at 4°C. G-10 Sephadex: add 100 g Sephadex G-10 (Pharmacia, Uppsala, Sweden) to 400 mL dd H₂O and allow to swell at room temperature for 2 hours. Decant fines and wash 3 times. Add NaN₃ and qs with dd H₂O to 500 mLs and store at 4°C.
Assemble Columns: Straw column, filter frit and tip (Kontes, SP 420160-000, 420162-002). Lowsorb tubes (Nunc) used in binding reaction. THP.1 cytosol spun at 15000 rpm for 5 min to clarify. THP.1 cytosol prepared by hypnotic treatment of cells and lysis by decompression in nitrogen. Nuclei and membrane fragments removed by differential centrifugation (10,000 g for 1 hour and 100,000 g for 1 hour).
Compounds: Non-radioactive Compound I with corresponding EtOH control (dilutions made in incubation buffer) and ³H-Compound I (dilutions in incubation buffer)

METHOD:
A. Column Preparation
   1. Begin 30 min before anticipated elution of reaction mixture.
   2. Add 3 mL of G-10 slurry to column for bed vol of 1.5 ml.
   3. Rinse with 7 mL elution buffer (fill to top of column)
   4. Cut columns down to size.
B. Sample Incubation
   1. 15 min incubation at 4°C.
   2. Binding reaction mixture; 100 µL cytosol, 10 uL cold Compound I or EtOH control, 10 µL ³H-Compound I (molar concentration depends on nature of study).
   3. "Free" control = 100 µL incubation buffer in lieu of cytosol preparation.
C. Sample Elution
   1. Elute at 4°C.
   2. Add total reaction volume to G-10 column.
   3. Add 400 µL elution buffer to column and discard eluate.
   4. Add 500 µL elution buffer to column, collecting eluted volume in 20 ml scintillation vial.
   5. Add 15 mL Ready Safe scintillation fluid.
   6. Vortex and count in liquid scintillation counter for 5 minutes. Include a "total input counts control" (10 µL of labeled ligand).
D. Data Analysis
   1. Plot DPMS as ouptut in graphic form and analyze by regression analysis and "Lundon ligand binding" software for the determination of IC 50 and Kd/Ki respectively.
   2. Rank order the IC50s of the tested compounds in the bioassay and compare to that generated by the binding assay and establish a correlation curve.

The binding assay was further validated by the following criteria : THP.1 cytosol demonstrated saturable and specific binding of the radiolabeled compound.

### Preparation of 4-(Fluorophenyl)-2-(4-hydroxyphenyl-3,5-t₂)-5-(4-pyridyl)imidazole, (Compound I).

A 2.9 mg (0.0059 mmol) portion of 2-(3,5-Dibromo-4-hydroxyphenyl)-4-(4-fluorophenyl)-5-(4-pyridyl)imidazole, Compound I(p), was dissolved in 0.95 mL of dry DMF and 0.05 mL of triethylamine in a 2.4 mL round bottom flask equipped with a small magnetic stirring bar. A 1.7 mg portion of 5% Pd/C (Engelhard lot 28845) was added, and the flask was attached to the stainless steel tritium manifold. The mixture was degassed through four freeze-pump-thaw cycles, then tritium gas (5.3 Ci, 0.091 mmol) was introduced. The reaction mixture was allowed to warm to room temperature and was stirred vigorously for 20h. The mixture was frozen in liquid nitrogen, the remaining tritium gas (2.4 Ci) was removed, and the flask was removed from the manifold. The reaction mixture was transferred, using 3 x 1 mL of methanol as rinsings, into a 10 mL round bottom flask, and the solvents were removed by static vacuum transfer. A 1.5 mL portion of methanol was added to the residue, then removed by static vacuum transfer. The latter process was repeated. Finally, the residue was suspended in 1.5 mL of ethanol and filtered through a syringe-tip Millipore filter (0.45 micron), along with 3 x ca. 1 mL ethanol rinsings. The total filtrate volume was determined to be 3.9 mL, and the total radioactivity, 94.2 mCi. Solution was determined to be 3.9 mL, and the total radioactivity, 94.2 mCi. HPLC analysis of filtrate (Partisil 5 ODS-3, 4.6 mm I.D. x 25 cm, 1 mL/min of 70:30:01 water/acetonitrile/trifluoroacetic acid, Radiomatic Flo-One Beta radio detector with 3 mL/min of Ecoscint-H cocktail through a 0.75 mL cell) showed the presence of Compound I (Rₜ = 60 min. ca. 37% of total radioactivity), and a discrete intermediate presumed to be the monobromo derivative Compound Ia (Rₜ = 11.8 min, ca. 9%).

The filtrate solution was evaporated to near dryness with a stream of nitrogen, and the residue was dissolved in about 1.2 mL of the HPLC mobile phase. The solution was separated by HPLC as shown below, and the peaks corresponding to Compounds I and Ia and SB collected separately.

| HPLC Method | |
|---|---|
| Column | Altex Ultrasphere |
| | 10 mm I.D. x 25 cm |
| Mobile Phase | 70:30:0.1 |
| | water/acetonitrile/trifluoroacetic acid |
| Flow Rate | 5 mL/min |
| UV detection | 210nm |
| Injection Volumes | 0.05 - 0.4 m: |
| Retention Times | 7.8 min Compound I |
| | 24 min Compound Ia |

The pooled Compound I fractions totaled 32 mL in volume and the radioactive concentration was 1.52 mCi/mL (total 48.6 m Ci). The pooled SB Compound Ia [³H] fractions (totaling 10.1 mCi) were evaporated to dryness and the residue was transferred quantitatively into a glass vial using 3.8 mL of absolute ethanol for further analysis.

An 8 mL (12.2 mCi) portion of Compound I was evaporated to dryness *in vacuo* at <35°C, then redissolved in 0.5 mL of mobile phase. The whole volume was injected into the HPLC system described above, and the appropriate peak was collected. Evaporation of the collected eluate *in vacuo* at <35°C and transfer of the yellow residue into a vial with absolute ethanol provided a solution (3.8 mL, 2.44 mCi/mL) of Compound I. The portion of this solution used for NMR analyses was first evaporated to dryness using stream of nitrogen then taken up in CD₃OD.

### Analysis of 4-(4-Fluorophenyl)-2-(4-hydroxyphenyl-3,5-t₂)-5-(4-pyridyl)imidazole, Compound I.

| Radiochemical Purity by HPLC | |
|---|---|
| Method | |
| Column | Ultrasphere Octyl, 5mm, 4.6 mm |
| | I.D. x 25 cm, Beckman |
| Mobile Phase | 350:150:0.5(v/v/v) |
| | water/acetonitrile/trifluoroacetic acid |
| Flow Rate | 1.0 mL/min |
| Mass detection | UV at 210 nm |
| Radioactivity detection | Ramona-D radioactivity flow detector |
| Scintillator | Tru-Count (Tru-Lab Supply Co.) |
| Flow rate | 5.0 mL/min |
| Cell volume | 0.75 mL |
| Retention time | 7.7 min |
| Result | 98.7 |

| Radioactive Concentration by Scintillation Counting | |
|---|---|
| Method | |
| Scintillator | Ready Safe (Beckman Instruments, Inc.) |
| Instrument | TM Analytic model 6881 |
| Efficiency | Automated DPM calculation from quench curve |
| Result | 2.44 mCi/mL |

| Specific Activity by Mass Spectrometry | | |
|---|---|---|
| Method | CI-MS, NH₃ reagent gas | |
| Result | 20.0 Ci/mmol | |
| | ³H Distribution: | |
| | Unlabeled | 44% |
| | Single Label | 43% |
| | Double Label | 13% |

| ³H NMR⁹ | |
|---|---|
| Method | |
| Instrument | Brunker AM 400 |
| Experiment | Proton decoupled ³H NMR |
| | Proton non-decoupled ³H NMR |
| | Proton non-decoupled ³H NMR |
| Peak Referencing | Solvent Peak of methanol • 3.3 |
| Solvent | Methanol-d₄ |
| Result | Tritium is incorporated exclusively on the carbon atoms ortho to aromatic hydroxyl group |

| Analytical Summary | |
|---|---|
| Assay | Result |
| Radiochemical purity determined by HPLC | 98.7% |
| Radioactivity concentration determined by scintillation counting | 2.44 mCi/mL |
| | |
| Specific activity determined by mass spectrometry | 20.0 Ci/mmol |
| ³H NMR | agrees with the |
| | proposed structure |

Representative compounds of Formula (I), Examples 1 to 77, but for the compound of example 2- not tested and compound of Example 72, have all demonstrated a positive inhibitory activity in this binding assay.

The above description fully discloses the invention including preferred embodiments thereof. Modifications and improvements of the embodiments specifically disclosed herein are within the scope of the following claims. Without further elaboration, it is believed that one skilled in the are can, using the preceding description, utilize the present invention to its fullest extent. Therefore the Examples herein are to be construed as merely illustrative and not a limitation of the scope of the present invention in any way. The embodiments of the invention in which an exclusive property or privilege is claimed are defined as follows.

## Claims

**1.** A compound represented by the formula : 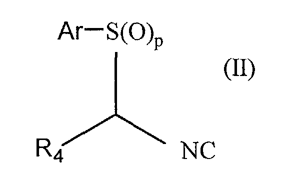 wherein p is 0, 1 or 2;
Ar is an optionally substituted phenyl group;
R₄ is phenyl, naphth-1-yl or naphth-2-yl which is optionally substituted by one or two substituents, each of which is independently selected, and which, for a 4-phenyl, 4-naphth-1-yl, 5-naphth-2-yl or 6-naphth-2-yl substituent, is halogen, cyano, nitro, C(Z)NR₇R₁₇, C(Z)OR₁₆, (CR₁₀R₂₀)ₘCOR₁₂, SR₅, SOR₅, OR₁₂, halo-substitutedC₁₋₄ alkyl, C₁₋₄ alkyl, ZC(Z)R₁₂, NR₁₀C(Z)R₁₆, or (CR₁₀R₂₀)ₘNR₁₀R₂₀ and which, for other positions of substitution, is halogen, cyano, C(Z)NR₁₃R₁₄, C(Z)OR₃, (CR₁₀R₂₀)ₘCOR₃, S(O)ₘR₃, OR₃, halo-substitutedC₁₋₄ alkyl, C₁₋₄ alkyl, (CR₁₀R₂₀)ₘNR₁₀C(Z)R₃, NR₁₀S(O)ₘR₈, NR₁₀S(O)ₘ'NR₇R₁₇, ZC(Z)R₃ or (CR₁₀R₂₀)ₘNR₁₃R₁₄;
n is an integer having a value of 1 to 10;
m is 0, or the integer 1 or 2;
m' is 1 or 2,
Z is oxygen or sulfur;
R₃ is heterocyclyl, heterocyclylC₁₋₁₀ alkyl or R₈;
R₅ is hydrogen, C₁₋₄ alkyl, C₂₋₄ alkenyl, C₂₋₄ alkynyl or NR₇R₁₇, excluding the moeities SR₅ being SNR₇R₁₇ and SOR₅ being SOH;
R₇ and R₁₇ is each independently selected from hydrogen or C₁₋₄ alkyl or R₇ and R₁₇ together with the nitrogen to which they are attached form a heterocyclic ring of 5 to 7 members which ring optionally contains an additional heteroatom selected from oxygen, sulfur or NR₁₅;
R₈ is C₁₋₁₀ alkyl, halo-substituted C₁₋₁₀ alkyl, C₂₋₁₀ alkenyl, C₂₋₁₀ alkynyl, C₃₋₇ cycloalkyl, C₅₋₇ cycloalkenyl, aryl, arylC₁₋₁₀ alkyl, heteroaryl, heteroarylC₁₋₁₀ alkyl, (CR₁₀R₂₀)ₙOR₁₁, (CR₁₀R₂₀)ₙS(O)ₘR₁₈, (CR₁₀R₂₀)ₙNHS(O)₂R₁₈, (CR₁₀R₂₀)ₙNR₁₃R₁₄; wherein the aryl, arylalkyl, heteroaryl, heteroaryl alkyl may be optionally substituted by halogen; hydroxy; hydroxy substituted C₁₋₁₀alkyl; C₁₋₁₀ alkoxy; S(O)m alkyl; NR₇R₁₇; C₁₋₁₀ alkyl, cycloalkyl, cycloalkyl alkyl group; halosubstituted C₁₋₁₀ alkyl; optionally substituted aryl; or optionally substituted arylalkyl, and wherein the optionally substituted aryl or arylalkyl moieties may be substituted one or two times by halogen; hydroxy; hydroxy substituted alkyl; C₁₋₁₀ alkoxy; S(O)ₘ alkyl; NR₇R₁₇; alkyl, or CF₃;
R₉ is hydrogen, C(Z)R₁₁ or optionally substituted C₁₋₁₀ alkyl, S(O)₂R₁₈, optionally substituted aryl or optionally substituted aryl-C₁₋₄ alkyl, and wherein the optional substituents for the alkyl, aryl and aryl alkyl groups are halogen; hydroxy; hydroxy substituted C₁₋₁₀alkyl; C₁₋₁₀ alkoxy; S(O)m alkyl; NR₇R₁₇; C₁₋₁₀ alkyl, cycloalkyl, cycloalkyl alkyl group; halosubstituted C₁₋₁₀ alkyl; optionally substituted aryl, or optionally substituted arylalkyl, wherein the optionally substituted aryl or arylalkyl moieties may be substituted one or two times by halogen; hydroxy; hydroxy substituted alkyl; C₁₋₁₀ alkoxy; S(O)ₘ alkyl; NR₇R₁₇; alkyl, or CF₃;
R₁₀ and R₂₀ is each independently selected from hydrogen or C₁₋₄ alkyl;
R₁₁ is hydrogen, C₁₋₁₀ alkyl, C₃₋₇ cycloalkyl, heterocyclyl, heterocyclyl C₁₋₁₀alkyl, aryl, arylC₁₋₁₀ alkyl, heteroaryl or heteroarylC₁₋₁₀ alkyl;
R₁₂ is hydrogen or R₁₆;
R₁₃ and R₁₄ is each independently selected from hydrogen or optionally substituted C₁₋₄ alkyl, optionally substituted aryl or optionally substituted aryl-C₁₋₄ alkyl, or together with the nitrogen to which they are attached form a heterocyclic ring of 5 to 7 members which ring optionally contains an additional heteroatom selected from oxygen, sulfur or NR₉; and wherein the optional substituents for the alkyl, aryl and aryl alkyl groups are halogen; hydroxy; hydroxy substituted C₁₋₁₀alkyl; C₁₋₁₀ alkoxy; S(O)m alkyl; NR₇R₁₇; C₁₋₁₀ alkyl, cycloalkyl, cycloalkyl alkyl group; halosubstituted C₁₋₁₀ alkyl; optionally substituted aryl, or optionally substituted arylalkyl, and wherein the optionally substituted aryl or arylalkyl moieties may be substituted one or two times by halogen; hydroxy; hydroxy substituted alkyl; C₁₋₁₀ alkoxy; S(O)ₘ alkyl; NR₇R₁₇ group; alkyl; or CF₃;
R₁₅ is R₁₀ or C(Z)-C₁₋₄ alkyl;
R₁₆ is C₁₋₄ alkyl, halo-substituted-C₁₋₄ alkyl, or C₃₋₇ cycloalkyl;
R₁₈ is C₁₋₁₀ alkyl, C₃₋₇ cycloalkyl, heterocyclyl, aryl, arylalkyl, heterocyclyl, heterocyclyl-C₁₋₁₀alkyl, heteroaryl or heteroarylalkyl; provided that when ArS(0)p is tosyl, R₄ is not unsubstituted phenyl.

**2.** A compound of the Formula (II) according to Claim 1 wherein p is 0.

**3.** A compound of the Formula (II) according to Claim 1 wherein p is 2.

**4.** The compound according to Claim 1 wherein the R₄ is a substituted phenyl.

**5.** The compound according to Claim 4 wherein the phenyl is monosubstituted in the 3-, or 4-position, or is disubstituted in the 3,4-position.

**6.** The compound according to Claim 4 wherein the phenyl is substituted by halogen, trifluromethyl, SR₅ or S(O)R₅.

**7.** The compound according to Claim 6 wherein R₅ is a C₁₋₂ alkyl.

**8.** The compound according to Claim 1 wherein R₄ is phenyl substituted by CF₃.

**9.** The compound according to Claim 1 wherein R₄ is phenyl substituted by chloro or fluoro.

**11.** The compound according to Claim 1 which is:
4-fluorophenyl-t tolylthiomethylisocyanide;
3-methylthiophenyl-(tolylthio)methylisocyanide ;
4-Fluorophenyl-tosylmethylisocyanide;
3-chlorophenyl-tolylthiomethylisocyanide;
4-trifluoromethylphenyl-tolylthiomethylisocyanide;
3-trifluoromethylphenyl-tolylthiomethylisocyanide;
3,4-dichlorophenyl-tolylthiomethylisocyanide; or
3,5-bistrifluoromethylphenyl-tolylthiomethylisocyanide.

**12.** A process for producing a compound of Formula (II) according to Claim 1 which process comprises dehydrating a compound of Formula (IV) 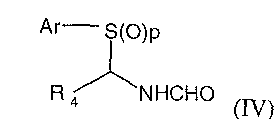 wherein Ar, R4 and p are as defined above for Formula (II), to produce a compound of Formula (II).

**13.** The process according to Claim 12 which process produces:
4-fluorophenyl-t tolylthiomethylisocyanide;
3-methylthiophenyl-(tolylthio)methylisocyanide ;
4-Fluorophenyl-tosylmethylisocyanide;
3-chlorophenyl-tolylthiomethylisocyanide;
4-trifluoromethylphenyl-tolylthiomethylisocyanide;
3-trifluoromethylphenyl-tolylthiomethylisocyanide;
3,4-dichlorophenyl-tolylthiomethylisocyanide; or
3,5-bistrifluoromethylphenyl-tolylthiomethylisocyanide.

**14.** The process according to Claim 12 wherein the compound of Formula (IV) is:
4-fluorophenyl-tolylthiomethylformamide;
N-[3-methylthiophenyl-(tolylthio)methyl]formamide ;
4 -Fluorophenyl-tosylmethylformamide;
3-chlorophenyl-tolylthiomethylformamide;
4-trifluoromethylphenyl-tolylthiomethylformamide;
3-trifluoromethylphenyl-tolylthiomethylformamide;
3,4-dichlorophenyl-tolylthiomethylformamide; or
3,5-bistrifluoromethylphenyl-tolylthiomethylformamide.

**15.** A process for producing a compound according to Claim 1, wherein p is 2 which process comprises reacting in the presence of a strong base a compound of the formula
R₄CH₂NC
wherein R₄ is as defined for Formula (II), with a compound of the formula ArSO₂L₁ wherein R₄ and Ar are as defined for Formula (II), and L₁ is a leaving group

**16.** The process according to Claim 15 wherein the strong base is an alkyl lithium reagent.

**17.** The process according to Claim 16 wherein the alkyl lithium reagent is butyl lithium or lithium diisopropylamide.
